# EUROPEAN PATENT SPECIFICATION

(11) **EP 3 793 587 B1**
(45) Date of publication and mention of the grant of the patent: **02.07.2025**
(21) Application number: 19724825.5
(22) Date of filing: 17.05.2019
(51) Int. Cl.: A61K 38/29, A61P 5/18, A61K 47/60

(54) **STARTING DOSE OF PTH CONJUGATES**
ANFANGSDOSIS VON PTH-KONJUGATEN
DOSE DE DÉPART DE CONJUGUÉS DE PTH

(30) Priority: 18.05.2018 EP 18173155
(43) Date of publication of application: 24.03.2021
(73) Proprietor: Ascendis Pharma Bone Diseases A/S, 2900 Hellerup (DK)
(72) Inventor: SPROGØE, Kennett, 2900 Hellerup (DK); KARPF, David Brian, Palo Alto, CA 94304 (US); STRANGE, Claus, 2900 Hellerup (DK)
(74) Representative: Büchel, Edwin
(86) International application number: PCT/EP2019/062773
(87) International publication number: WO 2019/219896

(56) References cited:
- WO-A1-2017/148883
- WO-A1-2018/060310
- WO-A1-2018/060311
- WO-A1-2018/060312
- WO-A2-2005/115441
- WO-A2-2009/095479
- ANROOPB NAIR ET AL: "A simple practice guide for dose conversion between animals and human", JOURNAL OF BASIC AND CLINICAL PHARMACY, vol. 7, no. 2, 1 May 2016 (2016-05-01), India, pages 27, XP055627116, ISSN: 0976-0105, DOI: 10.4103/0976-0105.177703
- NA D H ET AL: "Capillary electrophoretic characterization of PEGylated human parathyroid hormone with matrix-assisted laser desorption/ionization time-of-flight mass spectrometry", ANALYTICAL BIOCHEMISTRY, ELSEVIER, AMSTERDAM, NL, vol. 331, no. 2, 15 August 2004 (2004-08-15), pages 322 - 328, XP004521373, ISSN: 0003-2697, DOI: 10.1016/J.AB.2004.04.036

## Description

The present invention relates to a PTH conjugate, in which a PTH moiety is reversibly conjugated to a polymeric moiety or a pharmaceutically acceptable salt thereof or a pharmaceutical composition comprising said PTH conjugate or pharmaceutically acceptable salt thereof for use in the treatment, control, delay or prevention of a disease that can be treated, controlled, delayed or prevented with PTH, wherein the starting dose ranges from 0.8 to 4.9 nmol/day.

Hypoparathyroidism (HP) is a rare disorder of mineral metabolism. The clinical presentation may include muscle cramping, spasms, or tetany and, rarely, life-threatening events such as seizure or laryngospasm. Etiology is often neck surgery in adults or genetic disorders in children.

Conventional therapy with vitamin D analogs and calcium can normalize serum calcium, via increased intestinal absorption, but does not restore PTH-dependent renal calcium reabsorption or correct diminished bone turnover. Hypercalciuria and hyperphosphatemia often results, even when serum calcium is maintained at below-normal levels. Chronic hypercalciuria can cause irreversible renal damage and renal failure, and sustained hyperphosphatemia, resulting in an increased CxP product, can cause ectopic calcification in the renal parenchyma, the vascular system, the lens of the eye, and the basal ganglia of the brain, leading to additional potential morbidity and mortality. To reduce this risk, the vitamin D analog and calcium dosages are often reduced to maintain serum calcium at the lowest tolerated level. Consequently, many hypoparathyroid patients suffer varying degrees of hypocalcemia to avoid hypercalciuria-induced renal damage. (Clin Endocrinol Metab. 2012 Feb; 97(2): 391-399).

It is well known that overproduction of PTH produces a distinct pathophysiologic syndrome, hyperparathyroidism, characterized by hypercalcemia, hypercalciuria, hypophosphatemia, phosphaturia, and increases in osteoclastic bone resorption. Continuous exposure to supraphysiological PTH following continuous infusion replicates many of the symptoms of hyperparathyroidism. In this study, both PTH doses infused led to hypercalcemia with a resultant marked reduction in endogenous PTH (1-84) secretion, consistent with supraphysiological PTH doses. The suppression of endogenous PTH(1-84) secretion occurs in response to increases in serum calcium. The maximum suppression in response to hypercalcemia has been shown to be down to about 3-5 pg/ml in healthy volounteers, irrespective of baseline serum calcium values. (Journal of Bone and Mineral Research, Vol. 26, No. 9, September 2011, pp 2287-2297)

As one goal of HP therapy is to normalize serum calcium, supraphysiological levels of PTH should be avoided.

Single or twice-daily injection of PTH 1-34 can restore serum and urine calcium to the normal or near-normal range. Teriparatide has a short half-life of about 5 minutes after IV administration and 1 hour after subcutaneous administration due to prolonged absorbtion (Forteo Prescribing Information). Better results from twice-daily dosing can be explained by the short half-life of PTH(1-34), whose calcium-normalizing effects do not last a full 24 h after single administration.

In 2015, Natpara, PTH(1-84), was approved for once-daily subcutaneous injection as an adjunct to vitamin D and calcium in patients with hypoparathyroidism. While this represents an important advance in the treatment of the disease, Natpara has not demonstrated an ability to reduce incidences of hypercalcemia, hypocalcemia, or hypercalciuria relative to conventional therapy in treated patients, likely due to its short half-life (about 5 minutes IV; about 3 hours after subcutaneous administration).

As such, there is a high need for improved PTH based therapies for hypoparathyroidism that can maintain PTH levels and serum calcium in the physiological range. One such approach is to provide continuous exposure to PTH. For example, to facilitate more physiological PTH levels, clinical studies have been conducted with PTH(1-34) administered by pump delivery in comparison with twice-daily injections. Pump delivery ofPTH(1-34) achieved simultaneous normalization of markers of bone turnover, serum calcium, serum phosphate, serum magnesium, and urine calcium excretion.

However, the requirement to be reliant on an infusion pump is cumbersome for patients, and it would be a significant improvement if a continuous infusion-like exposure to PTH could be achieved with a single daily injection.

In order to address this issue reversible polymer conjugates of PTH were developed (WO2017/148883A1), which provide a sustained continuous release of PTH with daily injections. As both hypo- and hypercalcemia is associated with significant morbidity, it is paramount that the starting dose of such daily therapy is properly selected to ensure that patients receive both a clinical benefit while reducing the risk of adverse effects. So far, no information regarding suitable and safe starting doses for such PTH conjugates is available.

Thus, there is a need for identifying a safe and efficacious starting dose of a long acting PTH conjugate. This is the objective of the present invention.

Document WO 2017/148883 discloses in examples 16-18 PTH conjugates with PEG and having a linker according to the present claims and their use in therapy. Pharmacokinetic studies are performed in monkeys in examples 26, 27 and pharmacodynamic studies in rats in example 28.

This object is achieved with a PTH conjugate, in which a PTH moiety is reversibly conjugated to a polymeric moiety, or a pharmaceutically acceptable salt thereof or a pharmaceutical composition comprising said PTH conjugate or pharmaceutically acceptable salt thereof for use in the treatment of hypoparathyroidism in a human patient, wherein the starting dose ranges from 0.8 to 4.9 nmol/day and wherein the PTH conjugate is of formula (IIf-i). In certain embodiments the starting dose ranges from 0.8 to 3.9 nmol/day.

It was surprisingly found that a dose in the range of 0.8 to 4.9 nmol/day was able to induce suppression of endogenous PTH(1-84) secretion, while not inducing hypercalcemia and that a dose in the range of 0.8 to 3.9 nmol/day was able to increase serum calcium, while not completely suppressing endogenous PTH(1-84) secretion.

Within the present invention the terms are used having the meaning as follows.

As used herein the term "starting dose" refers to the dose of the PTH conjugate of the present invention that is administered to a patient when first initiating therapy with a PTH conjugate, i.e. such patient has not previously received a dose of said PTH conjugate. It is understood, that such starting dose may be continued for a period of time, such as several weeks or months, to allow the patient to stabilize and adjust other medication, such as oral calcium and active vitamin D.

The term "PTH" according to the present invention refers to the PTH polypeptide of SEQ ID NO:51:
SEQ ID NO:51 (PTH 1-34)
SVSEIQLMHNLGKHLNSMERVEWLRKKLQDVHNF

Also disclosed for the term "PTH" is a polypeptide sequence selected from the group consisting of:
SEQ ID NO:1 (PTH 1-84) 
SVSEIQLMHNLGKHLNSMERVEWLRKKLQDVHNFVALGAPLAPRDAGSQRPRKKE
DNVLVESHEKSLGEADKADVNVLTKAKSQ
SEQ ID NO:2 (PTH 1-83) 
SVSEIQLMHNLGKHLNSMERVEWLRKKLQDVHNFVALGAPLAPRDAGSQRPRKKE
DNVLVESHEKSLGEADKADVNVLTKAKS
SEQ ID NO:3 (PTH 1-82) 
SVSEIQLMHNLGKHLNSMERVEWLRKKLQDVHNFVALGAPLAPRDAGSQRPRKKE
DNVLVESHEKSLGEADKADVNVLTKAK
SEQ ID NO:4 (PTH 1-81) 
SVSEIQLMHNLGKHLNSMERVEWLRKKLQDVHNFVALGAPLAPRDAGSQRPRKKE
DNVLVESHEKSLGEADKADVNVLTKA
SEQ ID NO:5 (PTH 1-80) 
SVSEIQLMHNLGKHLNSMERVEWLRKKLQDVHNFVALGAPLAPRDAGSQRPRKKE
DNVLVESHEKSLGEADKADVNVLTK
SEQ ID NO:6 (PTH 1-79) 
SVSEIQLMHNLGKHLNSMERVEWLRKKLQDVHNFVALGAPLAPRDAGSQRPRKKE
DNVLVESHEKSLGEADKADVNVLT
SEQ ID NO:7 (PTH 1-78) 
SVSEIQLMHNLGKHLNSMERVEWLRKKLQDVHNFVALGAPLAPRDAGSQRPRKKE
DNVLVESHEKSLGEADKADVNVL
SEQ ID NO:8 (PTH 1-77) 
SVSEIQLMHNLGKHLNSMERVEWLRKKLQDVHNFVALGAPLAPRDAGSQRPRKKE
DNVLVESHEKSLGEADKADVNV
SEQ ID NO:9 (PTH 1-76) 
SVSEIQLMHNLGKHLNSMERVEWLRKKLQDVHNFVALGAPLAPRDAGSQRPRKKE
DNVLVESHEKSLGEADKADVN
SEQ ID NO:10 (PTH 1-75) 
SVSEIQLMHNLGKHLNSMERVEWLRKKLQDVHNFVALGAPLAPRDAGSQRPRKKE
DNVLVESHEKSLGEADKADV
SEQ ID NO:11 (PTH 1-74) 
SVSEIQLMHNLGKHLNSMERVEWLRKKLQDVHNFVALGAPLAPRDAGSQRPRKKE
DNVLVESHEKSLGEADKAD
SEQ ID NO:12 (PTH 1-73) 
SVSEIQLMHNLGKHLNSMERVEWLRKKLQDVHNFVALGAPLAPRDAGSQRPRKKE
DNVLVESHEKSLGEADKA
SEQ ID NO:13 (PTH 1-72) 
SVSEIQLMHNLGKHLNSMERVEWLRKKLQDVHNFVALGAPLAPRDAGSQRPRKKE
DNVLVESHEKSLGEADK
SEQ ID NO:14 (PTH 1-71) 
SVSEIQLMHNLGKHLNSMERVEWLRKKLQDVHNFVALGAPLAPRDAGSQRPRKKE
DNVLVESHEKSLGEAD
SEQ ID NO:15 (PTH 1-70) 
SVSEIQLMHNLGKHLNSMERVEWLRKKLQDVHNFVALGAPLAPRDAGSQRPRKKE
DNVLVESHEKSLGEA
SEQ ID NO:16 (PTH 1-69) 
SVSEIQLMHNLGKHLNSMERVEWLRKKLQDVHNFVALGAPLAPRDAGSQRPRKKE
DNVLVESHEKSLGE
SEQ ID NO:17 (PTH 1-68) 
SVSEIQLMHNLGKHLNSMERVEWLRKKLQDVHNFVALGAPLAPRDAGSQRPRKKE
DNVLVESHEKSLG
SEQ ID NO:18 (PTH 1-67) 
SVSEIQLMHNLGKHLNSMERVEWLRKKLQDVHNFVALGAPLAPRDAGSQRPRKKE
DNVLVESHEKSL
SEQ ID NO:19 (PTH 1-66) 
SVSEIQLMHNLGKHLNSMERVEWLRKKLQDVHNFVALGAPLAPRDAGSQRPRKKE
DNVLVESHEKS
SEQ ID NO:20 (PTH 1-65) 
SVSEIQLMHNLGKHLNSMERVEWLRKKLQDVHNFVALGAPLAPRDAGSQRPRKKE
DNVLVESHEK
SEQ ID NO:21 (PTH 1-64) 
SVSEIQLMHNLGKHLNSMERVEWLRKKLQDVHNFVALGAPLAPRDAGSQRPRKKE
DNVLVESHE
SEQ ID NO:22 (PTH 1-63) 
SVSEIQLMHNLGKHLNSMERVEWLRKKLQDVHNFVALGAPLAPRDAGSQRPRKKE
DNVLVESH
SEQ ID NO:23 (PTH 1-62) 
SVSEIQLMHNLGKHLNSMERVEWLRKKLQDVHNFVALGAPLAPRDAGSQRPRKKE
DNVLVES
SEQ ID NO:24 (PTH 1-61) 
SVSEIQLMHNLGKHLNSMERVEWLRKKLQDVHNFVALGAPLAPRDAGSQRPRKKE
DNVLVE
SEQ ID NO:25 (PTH 1-60) 
SVSEIQLMHNLGKHLNSMERVEWLRKKLQDVHNFVALGAPLAPRDAGSQRPRKKE
DNVLV
SEQ ID NO:26 (PTH 1-59) 
SVSEIQLMHNLGKHLNSMERVEWLRKKLQDVHNFVALGAPLAPRDAGSQRPRKKE
DNVL
SEQ ID NO:27 (PTH 1-58) 
SVSEIQLMHNLGKHLNSMERVEWLRKKLQDVHNFVALGAPLAPRDAGSQRPRKKE
DNV
SEQ ID NO:28 (PTH 1-57) 
SVSEIQLMHNLGKHLNSMERVEWLRKKLQDVHNFVALGAPLAPRDAGSQRPRKKE
DN
SEQ ID NO:29 (PTH 1-56) 
SVSEIQLMHNLGKHLNSMERVEWLRKKLQDVHNFVALGAPLAPRDAGSQRPRKKE
D
SEQ ID NO:30 (PTH 1-55)
   SVSEIQLMHNLGKHLNSMERVEWLRKKLQDVHNFVALGAPLAPRDAGSQRPRKKE
SEQ ID NO:31 (PTH 1-54)
   SVSEIQLMHNLGKHLNSMERVEWLRKKLQDVHNFVALGAPLAPRDAGSQRPRKK
SEQ ID NO:32 (PTH 1-53)
   SVSEIQLMHNLGKHLNSMERVEWLRKKLQDVHNFVALGAPLAPRDAGSQRPRK
SEQ ID NO:33 (PTH 1-52)
   SVSEIQLMHNLGKHLNSMERVEWLRKKLQDVHNFVALGAPLAPRDAGSQRPR
SEQ ID NO:34 (PTH 1-51)
   SVSEIQLMHNLGKHLNSMERVEWLRKKLQDVHNFVALGAPLAPRDAGSQRP
SEQ ID NO:35 (PTH 1-50)
   SVSEIQLMHNLGKHLNSMERVEWLRKKLQD VHNFVALGAPLAPRDAGSQR
SEQ ID NO:36 (PTH 1-49)
   SVSEIQLMHNLGKHLNSMERVEWLRKKLQDVHNFVALGAPLAPRDAGSQ
SEQ ID NO:37 (PTH 1-48)
   SVSEIQLMHNLGKHLNSMERVEWLRKKLQDVHNFVALGAPLAPRDAGS
SEQ ID NO:38 (PTH 1-47)
   SVSEIQLMHNLGKHLNSMERVEWLRKKLQDVHNFVALGAPLAPRDAG
SEQ ID NO:39 (PTH 1-46)
   SVSEIQLMHNLGKHLNSMERVEWLRKKLQDVHNFVALGAPLAPRDA
SEQ ID NO:40 (PTH 1-45)
   SVSEIQLMHNLGKHLNSMERVEWLRKKLQDVHNFVALGAPLAPRD
SEQ ID NO:41 (PTH 1-44)
   SVSEIQLMHNLGKHLNSMERVEWLRKKLQDVHNFVALGAPLAPR
SEQ ID NO:42 (PTH 1-43)
   SVSEIQLMHNLGKHLNSMERVEWLRKKLQDVHNFVALGAPLAP
SEQ ID NO:43 (PTH 1-42)
   SVSEIQLMHNLGKHLNSMERVEWLRKKLQDVHNFVALGAPLA
SEQ ID NO:44 (PTH 1-41)
   SVSEIQLMHNLGKHLNSMERVEWLRKKLQDVHNFVALGAPL
SEQ ID NO:45 (PTH 1-40)
   SVSEIQLMHNLGKHLNSMERVEWLRKKLQDVHNFVALGAP
SEQ ID NO:46 (PTH 1-39)
   SVSEIQLMHNLGKHLNSMERVEWLRKKLQDVHNFVALGA
SEQ ID NO:47 (PTH 1-38)
   SVSEIQLMHNLGKHLNSMERVEWLRKKLQDVHNFVALG
SEQ ID NO:48 (PTH 1-37)
   SVSEIQLMHNLGKHLNSMERVEWLRKKLQDVHNFVAL
SEQ ID NO:49 (PTH 1-36)
   SVSEIQLMHNLGKHLNSMERVEWLRKKLQDVHNFVA
SEQ ID NO:50 (PTH 1-35)
   SVSEIQLMHNLGKHLNSMERVEWLRKKLQDVHNFV
SEQ ID NO:52 (PTH 1-33)
   SVSEIQLMHNLGKHLNSMERVEWLRKKLQDVHN
SEQ ID NO:53 (PTH 1-32)
   SVSEIQLMHNLGKHLNSMERVEWLRKKLQDVH
SEQ ID NO:54 (PTH 1-31)
   SVSEIQLMHNLGKHLNSMERVEWLRKKLQDV
SEQ ID NO:55 (PTH 1-30)
   SVSEIQLMHNLGKHLNSMERVEWLRKKLQD
SEQ ID NO:56 (PTH 1-29)
   SVSEIQLMHNLGKHLNSMERVEWLRKKLQ
SEQ ID NO:57 (PTH 1-28)
   SVSEIQLMHNLGKHLNSMERVEWLRKKL
SEQ ID NO:58 (PTH 1-27)
   SVSEIQLMHNLGKHLNSMERVEWLRKK
SEQ ID NO:59 (PTH 1-26)
   SVSEIQLMHNLGKHLNSMERVEWLRK
SEQ ID NO:60 (PTH 1-25)
   SVSEIQLMHNLGKHLNSMERVEWLR
SEQ ID NO:61 (amidated PTH 1-84)
   SVSEIQLMHNLGKHLNSMERVEWLRKKLQDVHNFVALGAPLAPRDAGSQRPRKKE DNVLVESHEKSLGEADKADVNVLTKAKSQ; wherein the C-terminus is amidated
SEQ ID NO:62 (amidated PTH 1-83)
   SVSEIQLMHNLGKHLNSMERVEWLRKKLQDVHNFVALGAPLAPRDAGSQRPRKKE DNVLVESHEKSLGEADKADVNVLTKAKS; wherein the C-terminus is amidated
SEQ ID NO:63 (amidated PTH 1-82)
   SVSEIQLMHNLGKHLNSMERVEWLRKKLQDVHNFVALGAPLAPRDAGSQRPRKKE DNVLVESHEKSLGEADKADVNVLTKAK; wherein the C-terminus is amidated
SEQ ID NO:64 (amidated PTH 1-81)
   SVSEIQLMHNLGKHLNSMERVEWLRKKLQDVHNFVALGAPLAPRDAGSQRPRKKE DNVLVESHEKSLGEADKADVNVLTKA; wherein the C-terminus is amidated
SEQ ID NO:65 (amidated PTH 1-80)
   SVSEIQLMHNLGKHLNSMERVEWLRKKLQDVHNFVALGAPLAPRDAGSQRPRKKE DNVLVESHEKSLGEADKADVNVLTK; wherein the C-terminus is amidated
SEQ ID NO:66 (amidated PTH 1-79)
   SVSEIQLMHNLGKHLNSMERVEWLRKKLQDVHNFVALGAPLAPRDAGSQRPRKKE DNVLVESHEKSLGEADKADVNVLT; wherein the C-terminus is amidated
SEQ ID NO:67 (amidated PTH 1-78)
   SVSEIQLMHNLGKHLNSMERVEWLRKKLQDVHNFVALGAPLAPRDAGSQRPRKKE DNVLVESHEKSLGEADKADVNVL; wherein the C-terminus is amidated
SEQ ID NO:68 (amidated PTH 1-77)
   SVSEIQLMHNLGKHLNSMERVEWLRKKLQDVHNFVALGAPLAPRDAGSQRPRKKE DNVLVESHEKSLGEADKADVNV; wherein the C-terminus is amidated
SEQ ID NO:69 (amidated PTH 1-76)
   SVSEIQLMHNLGKHLNSMERVEWLRKKLQDVHNFVALGAPLAPRDAGSQRPRKKE DNVLVESHEKSLGEADKADVN; wherein the C-terminus is amidated
SEQ ID NO:70 (amidated PTH 1-75)
   SVSEIQLMHNLGKHLNSMERVEWLRKKLQDVHNFVALGAPLAPRDAGSQRPRKKE DNVLVESHEKSLGEADKADV; wherein the C-terminus is amidated
SEQ ID NO:71 (amidated PTH 1-74)
   SVSEIQLMHNLGKHLNSMERVEWLRKKLQDVHNFVALGAPLAPRDAGSQRPRKKE DNVLVESHEKSLGEADKAD; wherein the C-terminus is amidated
SEQ ID NO:72 (amidated PTH 1-73)
   SVSEIQLMHNLGKHLNSMERVEWLRKKLQDVHNFVALGAPLAPRDAGSQRPRKKE DNVLVESHEKSLGEADKA; wherein the C-terminus is amidated
SEQ ID NO:73 (amidated PTH 1-72)
   SVSEIQLMHNLGKHLNSMERVEWLRKKLQDVHNFVALGAPLAPRDAGSQRPRKKE DNVLVESHEKSLGEADK; wherein the C-terminus is amidated
SEQ ID NO:74 (amidated PTH 1-71)
   SVSEIQLMHNLGKHLNSMERVEWLRKKLQDVHNFVALGAPLAPRDAGSQRPRKKE DNVLVESHEKSLGEAD; wherein the C-terminus is amidated
SEQ ID NO:75 (amidated PTH 1-70)
   SVSEIQLMHNLGKHLNSMERVEWLRKKLQDVHNFVALGAPLAPRDAGSQRPRKKE DNVLVESHEKSLGEA; wherein the C-terminus is amidated
SEQ ID NO:76 (amidated PTH 1-69)
   SVSEIQLMHNLGKHLNSMERVEWLRKKLQDVHNFVALGAPLAPRDAGSQRPRKKE DNVLVESHEKSLGE; wherein the C-terminus is amidated
SEQ ID NO:77 (amidated PTH 1-68)
   SVSEIQLMHNLGKHLNSMERVEWLRKKLQDVHNFVALGAPLAPRDAGSQRPRKKE DNVLVESHEKSLG; wherein the C-terminus is amidated
SEQ ID NO:78 (amidated PTH 1-67)
   SVSEIQLMHNLGKHLNSMERVEWLRKKLQDVHNFVALGAPLAPRDAGSQRPRKKE DNVLVESHEKSL; wherein the C-terminus is amidated
SEQ ID NO:79 (amidated PTH 1-66)
   SVSEIQLMHNLGKHLNSMERVEWLRKKLQDVHNFVALGAPLAPRDAGSQRPRKKE DNVLVESHEKS; wherein the C-terminus is amidated
SEQ ID NO:80 (amidated PTH 1-65)
   SVSEIQLMHNLGKHLNSMERVEWLRKKLQDVHNFVALGAPLAPRDAGSQRPRKKE DNVLVESHEK; wherein the C-terminus is amidated
SEQ ID NO:81 (amidated PTH 1-64)
   SVSEIQLMHNLGKHLNSMERVEWLRKKLQDVHNFVALGAPLAPRDAGSQRPRKKE DNVLVESHE; wherein the C-terminus is amidated
SEQ ID NO:82 (amidated PTH 1-63)
   SVSEIQLMHNLGKHLNSMERVEWLRKKLQDVHNFVALGAPLAPRDAGSQRPRKKE DNVLVESH; wherein the C-terminus is amidated
SEQ ID NO:83 (amidated PTH 1-62)
   SVSEIQLMHNLGKHLNSMERVEWLRKKLQDVHNFVALGAPLAPRDAGSQRPRKKE DNVLVES; wherein the C-terminus is amidated
SEQ ID NO:84 (amidated PTH 1-61)
   SVSEIQLMHNLGKHLNSMERVEWLRKKLQDVHNFVALGAPLAPRDAGSQRPRKKE DNVLVE; wherein the C-terminus is amidated
SEQ ID NO:85 (amidated PTH 1-60)
   SVSEIQLMHNLGKHLNSMERVEWLRKKLQDVHNFVALGAPLAPRDAGSQRPRKKE DNVLV; wherein the C-terminus is amidated
SEQ ID NO:86 (amidated PTH 1-59)
   SVSEIQLMHNLGKHLNSMERVEWLRKKLQDVHNFVALGAPLAPRDAGSQRPRKKE DNVL; wherein the C-terminus is amidated
SEQ ID NO:87 (amidated PTH 1-58)
   SVSEIQLMHNLGKHLNSMERVEWLRKKLQDVHNFVALGAPLAPRDAGSQRPRKKE DNV; wherein the C-terminus is amidated
SEQ ID NO:88 (amidated PTH 1-57)
   SVSEIQLMHNLGKHLNSMERVEWLRKKLQDVHNFVALGAPLAPRDAGSQRPRKKE DN; wherein the C-terminus is amidated
SEQ ID NO:89 (amidated PTH 1-56)
   SVSEIQLMHNLGKHLNSMERVEWLRKKLQDVHNFVALGAPLAPRDAGSQRPRKKE D; wherein the C-terminus is amidated
SEQ ID NO:90 (amidated PTH 1-55)
   SVSEIQLMHNLGKHLNSMERVEWLRKKLQDVHNFVALGAPLAPRDAGSQRPRKKE; wherein the C-terminus is amidated
SEQ ID NO:91 (amidated PTH 1-54)
   SVSEIQLMHNLGKHLNSMERVEWLRKKLQDVHNFVALGAPLAPRDAGSQRPRKK; wherein the C-terminus is amidated
SEQ ID NO:92 (amidated PTH 1-53)
   SVSEIQLMHNLGKHLNSMERVEWLRKKLQDVHNFVALGAPLAPRDAGSQRPRK; wherein the C-terminus is amidated
SEQ ID NO:93 (amidated PTH 1-52)
   SVSEIQLMHNLGKHLNSMERVEWLRKKLQDVHNFVALGAPLAPRDAGSQRPR; wherein the C-terminus is amidated
SEQ ID NO:94 (amidated PTH 1-51)
   SVSEIQLMHNLGKHLNSMERVEWLRKKLQDVHNFVALGAPLAPRDAGSQRP; wherein the C-terminus is amidated
SEQ ID NO:95 (amidated PTH 1-50)
   SVSEIQLMHNLGKHLNSMERVEWLRKKLQDVHNFVALGAPLAPRDAGSQR; wherein the C-terminus is amidated
SEQ ID NO:96 (amidated PTH 1-49)
   SVSEIQLMHNLGKHLNSMERVEWLRKKLQDVHNFVALGAPLAPRDAGSQ; wherein the C-terminus is amidated
SEQ ID NO:97 (amidated PTH 1-48)
   SVSEIQLMHNLGKHLNSMERVEWLRKKLQDVHNFVALGAPLAPRDAGS; wherein the C-terminus is amidated
SEQ ID NO:98 (amidated PTH 1-47)
   SVSEIQLMHNLGKHLNSMERVEWLRKKLQDVHNFVALGAPLAPRDAG; wherein the C-terminus is amidated
SEQ ID NO:99 (amidated PTH 1-46)
   SVSEIQLMHNLGKHLNSMERVEWLRKKLQDVHNFVALGAPLAPRDA; wherein the C-terminus is amidated
SEQ ID NO:100 (amidated PTH 1-45)
   SVSEIQLMHNLGKHLNSMERVEWLRKKLQDVHNFVALGAPLAPRD; wherein the C-terminus is amidated
SEQ ID NO:101 (amidated PTH 1-44)
   SVSEIQLMHNLGKHLNSMERVEWLRKKLQDVHNFVALGAPLAPR; wherein the C-terminus is amidated
SEQ ID NO:102 (amidated PTH 1-43)
   SVSEIQLMHNLGKHLNSMERVEWLRKKLQDVHNFVALGAPLAP; wherein the C-terminus is amidated
SEQ ID NO:103 (amidated PTH 1-42)
   SVSEIQLMHNLGKHLNSMERVEWLRKKLQDVHNFV ALGAPLA; wherein the C-terminus is amidated
SEQ ID NO:104 (amidated PTH 1-41)
   SVSEIQLMHNLGKHLNSMERVEWLRKKLQDVHNFVALGAPL; wherein the C-terminus is amidated
SEQ ID NO:105 (amidated PTH 1-40)
   SVSEIQLMHNLGKHLNSMERVEWLRKKLQDVHNFV ALGAP; wherein the C-terminus is amidated
SEQ ID NO:106 (amidated PTH 1-39)
   SVSEIQLMHNLGKHLNSMERVEWLRKKLQDVHNFVALGA; wherein the C-terminus is amidated
SEQ ID NO:107 (amidated PTH 1-38)
   SVSEIQLMHNLGKHLNSMERVEWLRKKLQDVHNFVALG; wherein the C-terminus is amidated
SEQ ID NO:108 (amidated PTH 1-37)
   SVSEIQLMHNLGKHLNSMERVEWLRKKLQDVHNFVAL; wherein the C-terminus is amidated
SEQ ID NO:109 (amidated PTH 1-36)
   SVSEIQLMHNLGKHLNSMERVEWLRKKLQDVHNFVA; wherein the C-terminus is amidated
SEQ ID NO:110 (amidated PTH 1-35)
   SVSEIQLMHNLGKHLNSMERVEWLRKKLQDVHNFV; wherein the C-terminus is amidated
SEQ ID NO:111 (amidated PTH 1-34)
   SVSEIQLMHNLGKHLNSMERVEWLRKKLQDVHNF; wherein the C-terminus is amidated
SEQ ID NO:112 (amidated PTH 1-33)
   SVSEIQLMHNLGKHLNSMERVEWLRKKLQDVHN; wherein the C-terminus is amidated
SEQ ID NO:113 (amidated PTH 1-32)
   SVSEIQLMHNLGKHLNSMERVEWLRKKLQDVH; wherein the C-terminus is amidated
SEQ ID NO:114 (amidated PTH 1-31)
   SVSEIQLMHNLGKHLNSMERVEWLRKKLQDV; wherein the C-terminus is amidated
SEQ ID NO:115 (amidated PTH 1-30)
   SVSEIQLMHNLGKHLNSMERVEWLRKKLQD; wherein the C-terminus is amidated
SEQ ID NO:116 (amidated PTH 1-29)
   SVSEIQLMHNLGKHLNSMERVEWLRKKLQ; wherein the C-terminus is amidated
SEQ ID NO:117 (amidated PTH 1-28)
   SVSEIQLMHNLGKHLNSMERVEWLRKKL; wherein the C-terminus is amidated
SEQ ID NO:118 (amidated PTH 1-27)
   SVSEIQLMHNLGKHLNSMERVEWLRKK; wherein the C-terminus is amidated
SEQ ID NO:119 (amidated PTH 1-26)
   SVSEIQLMHNLGKHLNSMERVEWLRK; wherein the C-terminus is amidated
SEQ ID NO:120 (amidated PTH 1-25)
   SVSEIQLMHNLGKHLNSMERVEWLR; wherein the C-terminus is amidated
SEQ ID NO:121 (PTHrP)

AVSEHQLLHDKGKSIQDLRRRFFLHHLIAEIHTAEIRATSEVSPNSKPSPNTKNHPVRF
GSDDEGRYLTQETNKVETYKEQPLKTPGKKKKGKPGKRKEQEKKKRRTRSAWLDS
GVTGSGLEGDHLSDTSTTSLELDSRRH

As used herein, the term "random coil" refers to a peptide or protein adopting/having/forming, preferably having, a conformation which substantially lacks a defined secondary and tertiary structure as determined by circular dichroism spectroscopy performed in aqueous buffer at ambient temperature, and pH 7.4. Preferably, ambient temperature is about 20°C, i.e. between 18°C and 22°C, most preferably ambient temperature is 20°C.

As used herein the term "pharmaceutical composition" refers to a composition containing one or more active ingredients, for example a drug or a conjugate, here specifically the PTH conjugate of the present invention, and optionally one or more excipients, as well as any product which results, directly or indirectly, from combination, complexation or aggregation of any two or more of the ingredients of the composition, or from dissociation of one or more of the ingredients, or from other types of reactions or interactions of one or more of the ingredients. Accordingly, the pharmaceutical compositions of the present invention encompass any composition made by admixing one or more PTH conjugates of the present invention and optionally a pharmaceutically acceptable excipient.

As used herein the term "liquid composition" refers to a mixture comprising water-soluble PTH conjugate and one or more solvents, such as water.

As used herein, the term "dry composition" means that a pharmaceutical composition is provided in a dry form. Suitable methods for drying are spray-drying and lyophilization, i.e. freeze-drying. Such dry composition of for example the PTH conjugate of the present invention has a residual water content of a maximum of 10%, preferably less than 5% and more preferably less than 2%, determined according to Karl Fischer. Preferably, the pharmaceutical composition of the present invention is dried by lyophilization.

The term "drug" as used herein refers to a substance used in the treatment, cure, prevention, or diagnosis of a disease or used to otherwise enhance physical or mental well-being. If a drug is conjugated to another moiety, the moiety of the resulting product that originated from the drug is referred to as "biologically active moiety". The PTH conjugate of the present invention comprise a PTH moiety, which is released from the PTH conjugate in the form of the drug PTH.

It is understood that the PTH conjugates of the present invention are PTH produgs. As used herein the term "prodrug" refers to a conjugate comprising a biologically active moiety reversibly and covalently connected to a specialized protective group through a reversible linker moiety, also called prodrug linker moiety, which is a linker moiety comprising a reversible linkage with the biologically active moiety and wherein the specialized protective group alters or eliminates undesirable properties in the parent molecule. This also includes the enhancement of desirable properties in the drug and the suppression of undesirable properties. The specialized non-toxic protective group is referred to as "carrier". A prodrug releases the reversibly and covalently bound biologically active moiety in the form of its corresponding drug. In other words, a prodrug is a conjugate comprising a biologically active moiety, which is covalently and reversibly conjugated to a carrier moiety via a reversible linker moiety, which covalent and reversible conjugation of the carrier to the reversible linker moiety is either directly or through a spacer. Such conjugate releases the formerly conjugated biologically active moiety in its free form.

A "biodegradable linkage" or a "reversible linkage" is a linkage that is degradable, i.e. cleavable, for example by hydrolysis, in the absence of enzymes under physiological conditions (aqueous buffer at pH 7.4, 37°C) with a half-life ranging from one hour to two months, preferably from three hours to one month, even more preferably from 6 hours to two weeks. Accordingly, a stable linkage is a linkage having a half-life under physiological conditions (aqueous buffer at pH 7.4, 37°C) of more than two months.

Accordingly, a "reversible prodrug linker moiety" or "reversible linker moiety" is a moiety, which is covalently conjugated to a biologically active moiety, such as PTH, through a reversible linkage and is covalently conjugated to a carrier moiety, such as -Z, wherein the covalent conjugation to said carrier moiety is either directly or through a spacer moiety, such as -L²-. Preferably the linkage between -Z and -L²- is a stable linkage.

As used herein, the term "traceless prodrug linker" means a reversible prodrug linker, which upon cleavage releases the drug in its free form. As used herein, the term "free form" of a drug means the drug in its unmodified, pharmacologically active form.

As used herein, the term "excipient" refers to a diluent, adjuvant, or vehicle with which the therapeutic, such as a drug or the PTH conjugate of the present invention, is administered. Such pharmaceutical excipient can be sterile liquids, such as water and oils, including those of petroleum, animal, vegetable or synthetic origin, including peanut oil, soybean oil, mineral oil and sesame oil. Water is a preferred excipient when the pharmaceutical composition is administered orally. Saline and aqueous dextrose are preferred excipients when the pharmaceutical composition is administered intravenously. Saline solutions and aqueous dextrose and glycerol solutions are preferably employed as liquid excipients for injectable solutions. Suitable pharmaceutical excipients include starch, glucose, lactose, sucrose, mannitol, trehalose, gelatin, malt, rice, flour, chalk, silica gel, sodium stearate, glycerol monostearate, talc, sodium chloride, dried skim milk, glycerol, propylene, glycol, water and ethanol. The pharmaceutical composition, if desired, can also contain minor amounts of wetting or emulsifying agents, pH buffering agents, like, for example, acetate, succinate, tris, carbonate, phosphate, HEPES (4-(2-hydroxyethyl)-1-piperazineethanesulfonic acid), MES (2-(N-morpholino)ethanesulfonic acid), or can contain detergents, like Tween, poloxamers, poloxamines, CHAPS, Igepal, or amino acids like, for example, glycine, lysine, or histidine. These pharmaceutical compositions can take the form of solutions, suspensions, emulsions, tablets, pills, capsules, powders and sustained-release formulations. The pharmaceutical composition can be formulated as a suppository, with traditional binders and excipients such as triglycerides. Oral formulation can include standard excipients such as pharmaceutical grades of mannitol, lactose, starch, magnesium stearate, sodium saccharine, cellulose, magnesium carbonate, etc. Such compositions will contain a therapeutically effective amount of the drug or biologically active moiety, together with a suitable amount of excipient to provide the form for proper administration to the patient. The formulation should suit the mode of administration.

As used herein, the term "reagent" means a chemical compound, which comprises at least one functional group for reaction with the functional group of another chemical compound or drug. It is understood that a drug comprising a functional group (such as a primary or secondary amine or hydroxyl functional group) is also a reagent.

As used herein, the term "moiety" means a part of a molecule, which lacks one or more atom(s) compared to the corresponding reagent. If, for example, a reagent of the formula "H-X-H" reacts with another reagent and becomes part of the reaction product, the corresponding moiety of the reaction product has the structure "H-X-" or "-X-", whereas each "-" indicates attachment to another moiety. Accordingly, a biologically active moiety is released from the conjugates of the present invention as a drug.

It is understood that if the sequence or chemical structure of a group of atoms is provided which group of atoms is attached to two moieties or is interrupting a moiety, said sequence or chemical structure can be attached to the two moieties in either orientation, unless explicitly stated otherwise. For example, a moiety "-C(O)N(R)-" can be attached to two moieties or interrupting a moiety either as "-C(O)N(R)-" or as "-N(R)C(O)-". Similarly, a moiety 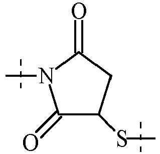 can be attached to two moieties or can interrupt a moiety either as

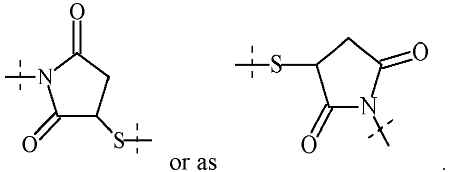

As used herein, the term "functional group" means a group of atoms, which can react with other groups of atoms. Functional groups include but are not limited to the following groups: carboxylic acid (-(C=O)OH), primary or secondary amine (-NH₂, -NH-), maleimide, thiol (-SH), sulfonic acid (-(O=S=O)OH), carbonate, carbamate (-O(C=O)N<), hydroxyl (-OH), aldehyde (-(C=O)H), ketone (-(C=O)-), hydrazine (>N-N<), isocyanate, isothiocyanate, phosphoric acid (-O(P=O)OHOH), phosphonic acid (-O(P=O)OHH), haloacetyl, alkyl halide, acryloyl, aryl fluoride, hydroxylamine, disulfide, sulfonamides, sulfuric acid, vinyl sulfone, vinyl ketone, diazoalkane, oxirane, and aziridine.

In case the conjugates of the present invention comprise one or more acidic or basic groups, the invention also comprises their corresponding pharmaceutically or toxicologically acceptable salts, in particular their pharmaceutically utilizable salts. Thus, the conjugates of the present invention comprising acidic groups can be used according to the invention, for example, as alkali metal salts, alkaline earth metal salts or as ammonium salts. More precise examples of such salts include sodium salts, potassium salts, calcium salts, magnesium salts or salts with ammonia or organic amines such as, for example, ethylamine, ethanolamine, triethanolamine or amino acids. Conjugates of the present invention comprising one or more basic groups, i.e. groups which can be protonated, can be present and can be used according to the invention in the form of their addition salts with inorganic or organic acids. Examples for suitable acids include hydrogen chloride, hydrogen bromide, phosphoric acid, sulfuric acid, nitric acid, methanesulfonic acid, p-toluenesulfonic acid, naphthalenedisulfonic acids, oxalic acid, acetic acid, tartaric acid, lactic acid, salicylic acid, benzoic acid, formic acid, propionic acid, pivalic acid, diethylacetic acid, malonic acid, succinic acid, pimelic acid, fumaric acid, maleic acid, malic acid, sulfaminic acid, phenylpropionic acid, gluconic acid, ascorbic acid, isonicotinic acid, citric acid, adipic acid, and other acids known to the person skilled in the art. For the person skilled in the art further methods are known for converting the basic group into a cation like the alkylation of an amine group resulting in a positively-charge ammonium group and an appropriate counterion of the salt. If the conjugates of the present invention simultaneously comprise acidic and basic groups, the invention also includes, in addition to the salt forms mentioned, inner salts or betaines (zwitterions). The respective salts can be obtained by customary methods, which are known to the person skilled in the art like, for example by contacting these conjugates with an organic or inorganic acid or base in a solvent or dispersant, or by anion exchange or cation exchange with other salts. The present invention also includes all salts of the conjugates of the present invention which, owing to low physiological compatibility, are not directly suitable for use in pharmaceuticals but which can be used, for example, as intermediates for chemical reactions or for the preparation of pharmaceutically acceptable salts.

The term "pharmaceutically acceptable" means a substance that does not cause harm when administered to a patient and preferably means approved by a regulatory agency, such as the EMA (Europe) and/or the FDA (US) and/or any other national regulatory agency for use in animals, preferably for use in humans.

As used herein the term "about" in combination with a numerical value is used to indicate a range ranging from and including the numerical value plus and minus no more than 20% of said numerical value, more preferably no more than 10% of said numerical value, even more preferably no more than 5% of said numerical value and most preferably no more than 2% of said numerical value. For example, the phrase "about 200" is used to mean a range ranging from and including 200 +/- 20%, i.e. ranging from and including 160 to 240; preferably 200 +/- 10%, i.e. ranging from and including 180 to 220; even more preferably ranging from and including 200 +/-5%, i.e. ranging from and including 190 to 210; and most preferably 200 +/-2%, i.e. ranging from and including 196 to 204. It is understood that a percentage given as "about 20%" does not mean "20% +/- 10%", i.e. ranging from and including 10 to 30%, but "about 20%" means ranging from and including 18 to 22%, i.e. plus and minus 10% of the numerical value which is 20.

As used herein, the term "polymer" means a molecule comprising repeating structural units, i.e. the monomers, connected by chemical bonds in a linear, circular, branched, crosslinked or dendrimeric way or a combination thereof, which may be of synthetic or biological origin or a combination of both. It is understood that a polymer may also comprise one or more other chemical group(s) and/or moiety/moieties, such as, for example, one or more functional group(s). Preferably, a soluble polymer has a molecular weight of at least 0.5 kDa, e.g. a molecular weight of at least 1 kDa, a molecular weight of at least 2 kDa, a molecular weight of at least 3 kDa or a molecular weight of at least 5 kDa. If the polymer is soluble, it preferable has a molecular weight of at most 1000 kDa, such as at most 750 kDa, such as at most 500 kDa, such as at most 300 kDa, such as at most 200 kDa, such as at most 100 kDa.

As used herein, the term "polymeric" means a reagent or a moiety comprising one or more polymer(s) or polymer moiety/moieties. A polymeric reagent or moiety may optionally also comprise one or more other moiety/moieties, which are preferably selected from the group consisting of:
- C₁₋₅₀ alkyl, C₂₋₅₀ alkenyl, C₂₋₅₀ alkynyl, C₃₋₁₀ cycloalkyl, 3- to 10-membered heterocyclyl, 8- to 11-membered heterobicyclyl, phenyl, naphthyl, indenyl, indanyl, and tetralinyl; and
- linkages selected from the group comprising 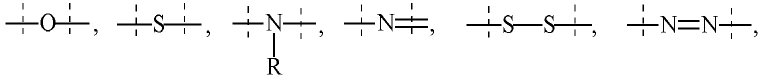 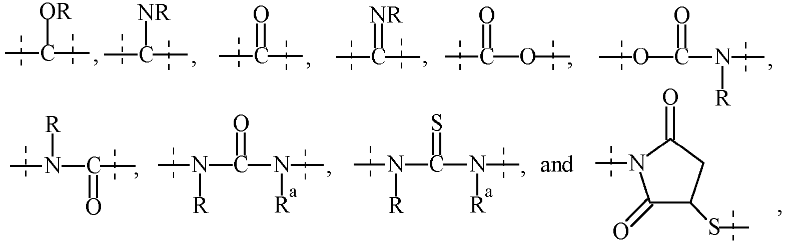 wherein
   dashed lines indicate attachment to the remainder of the moiety or reagent, and
   -R and -R^{a} are independently of each other selected from the group consisting of -H, methyl, ethyl, propyl, butyl, pentyl and hexyl.

The person skilled in the art understands that the polymerization products obtained from a polymerization reaction do not all have the same molecular weight, but rather exhibit a molecular weight distribution. Consequently, the molecular weight ranges, molecular weights, ranges of numbers of monomers in a polymer and numbers of monomers in a polymer as used herein, refer to the number average molecular weight and number average of monomers, i.e. to the arithmetic mean of the molecular weight of the polymer or polymeric moiety and the arithmetic mean of the number of monomers of the polymer or polymeric moiety.

Accordingly, in a polymeric moiety comprising "x" monomer units any integer given for "x" therefore corresponds to the arithmetic mean number of monomers. Any range of integers given for "x" provides the range of integers in which the arithmetic mean numbers of monomers lies. An integer for "x" given as "about x" means that the arithmetic mean numbers of monomers lies in a range of integers of x +/- 20%, preferably x +/- 10%, more preferably x +/- 5% and most preferably x +/- 2%.

As used herein, the term "number average molecular weight" means the ordinary arithmetic mean of the molecular weights of the individual polymers.

As used herein the term "water-soluble" with reference to a carrier means that when such carrier is part of the PTH conjugates of the present invention at least 1 g of the PTH conjugate comprising such water-soluble carrier can be dissolved in one liter of water at 20°C to form a homogeneous solution. Accordingly, the term "water-insoluble" with reference to a carrier means that when such carrier is part of the PTH conjugate of the present invention less than 1 g of the PTH conjugate comprising such water-insoluble carrier can be dissolved in one liter of water at 20°C to form a homogeneous solution.

As used herein, the term "PEG-based" in relation to a moiety or reagent means that said moiety or reagent comprises PEG. Preferably, a PEG-based moiety or reagent comprises at least 10% (w/w) PEG, such as at least 20% (w/w) PEG, such as at least 30% (w/w) PEG, such as at least 40% (w/w) PEG, such as at least 50% (w/w), such as at least 60 (w/w) PEG, such as at least 70% (w/w) PEG, such as at least 80% (w/w) PEG, such as at least 90% (w/w) PEG, such as at least 95%. The remaining weight percentage of the PEG-based moiety or reagent are other moieties preferably selected from the following moieties and linkages:
- C₁₋₅₀ alkyl, C₂₋₅₀ alkenyl, C₂₋₅₀ alkynyl, C₃₋₁₀ cycloalkyl, 3- to 10-membered heterocyclyl, 8- to 11-membered heterobicyclyl, phenyl, naphthyl, indenyl, indanyl, and tetralinyl; and
- linkages selected from the group comprising 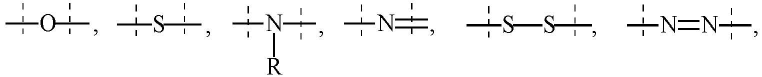 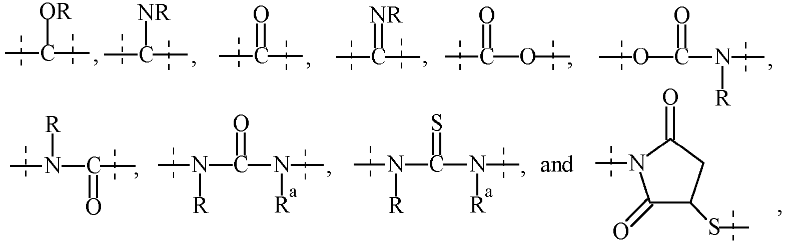 wherein
   dashed lines indicate attachment to the remainder of the moiety or reagent, and
   -R and -R^{a} are independently of each other selected from the group consisting of -H, methyl, ethyl, propyl, butyl, pentyl and hexyl.

As used herein, the term "PEG-based comprising at least X% PEG" in relation to a moiety or reagent means that said moiety or reagent comprises at least X% (w/w) ethylene glycol units (-CH₂CH₂O-), wherein the ethylene glycol units may be arranged blockwise, alternating or may be randomly distributed within the moiety or reagent and preferably all ethylene glycol units of said moiety or reagent are present in one block; the remaining weight percentage of the PEG-based moiety or reagent are other moieties preferably selected from the following moieties and linkages:
- C₁₋₅₀ alkyl, C₂₋₅₀ alkenyl, C₂₋₅₀ alkynyl, C₃₋₁₀ cycloalkyl, 3- to 10-membered heterocyclyl, 8- to 11-membered heterobicyclyl, phenyl, naphthyl, indenyl, indanyl, and tetralinyl; and
- linkages selected from the group comprising 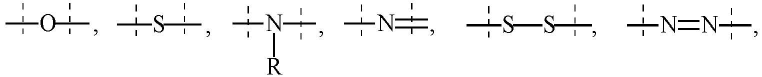 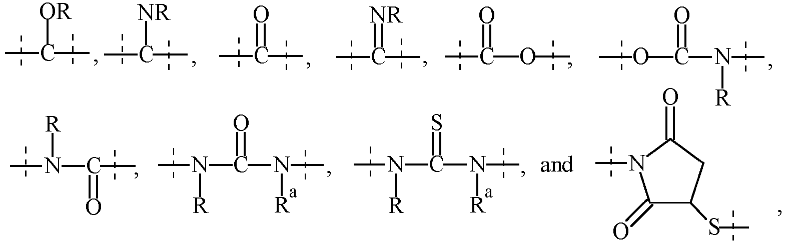 wherein
   dashed lines indicate attachment to the remainder of the moiety or reagent, and
   -R and -R^{a} are independently of each other selected from the group consisting of -H, methyl, ethyl, propyl, butyl, pentyl and hexyl.

The term "hyaluronic acid-based comprising at least X% hyaluronic acid" is used accordingly.

The term "substituted" as used herein means that one or more -H atom(s) of a molecule or moiety are replaced by a different atom or a group of atoms, which are referred to as "substituent".

Preferably, the one or more further optional substituents are independently of each other selected from the group consisting of halogen, -CN, -COOR^{x1}, -OR^{x1}, -C(O)R^{x1}, -C(O)N(R^{x1}R^{x1a}), -S(O)₂N(R^{x1}R^{x1a}), -S(O)N(R^{x1}R^{x1a}), -S(O)₂R^{x1}, -S(O)R^{x1}, -N(R^{x1})S(O)₂N(R^{x1a}R^{x1b}), -SR^{x1}, -N(R^{x1}R^{x1a}), -NO₂, -OC(O)R^{x1}, -N(R^{x1})C(O)R^{x1a}, -N(R^{x1})S(O)₂R^{x1a}, -N(R^{x1})S(O)R^{x1a}, -N(R^{x1})C(O)OR^{x1a}, -N(R^{x1})C(O)N(R^{x1a}R^{x1b}), -OC(O)N(R^{x1}R^{x1a}), -T⁰, C₁₋₅₀ alkyl, C₂₋₅₀ alkenyl, and C₂₋₅₀ alkynyl; wherein -T⁰, C₁₋₅₀ alkyl, C₂₋₅₀ alkenyl, and C₂₋₅₀ alkynyl are optionally substituted with one or more -R^{x2}, which are the same or different and wherein C₁₋₅₀ alkyl, C₂₋₅₀ alkenyl, and C₂₋₅₀ alkynyl are optionally interrupted by one or more groups selected from the group consisting of -T⁰-, -C(O)O-, -O-, -C(O)-, -C(O)N(R^{x3})-, -S(O)₂N(R^{x3})-, -S(O)N(R^{x3})-, -S(O)₂-, -S(O)-, -N(R^{x3})S(O)₂N(R^{x3a})-, -S-, -N(R^{x3})-, -OC(OR^{x3})(R^{x3a})-, -N(R^{x3})C(O)N(R^{x3a})-, and -OC(O)N(R^{x3})-;
-R^{x1}, -R^{x1a}, -R^{x1b} are independently of each other selected from the group consisting of -H, -T⁰, C₁₋₅₀ alkyl, C₂₋₅₀ alkenyl, and C₂₋₅₀ alkynyl; wherein -T⁰, C₁₋₅₀ alkyl, C₂₋₅₀ alkenyl, and C₂₋₅₀ alkynyl are optionally substituted with one or more -R^{x2}, which are the same or different and wherein C₁₋₅₀ alkyl, C₂₋₅₀ alkenyl, and C₂₋₅₀ alkynyl are optionally interrupted by one or more groups selected from the group consisting of -T⁰-, -C(O)O-, -O-, -C(O)-, -C(O)N(R^{x3})-, -S(O)₂N(R^{x3})-, -S(O)N(R^{x3})-; -S(O)₂-, -S(O)-, -N(R^{x3})S(O)₂N(R^{x3a})-, -S-, -N(R^{x3})-, -OC(OR^{x3})(R^{x3a})-, -N(R^{x3})C(O)N(R^{x3a})-, and -OC(O)N(R^{x3})-;
each T⁰ is independently selected from the group consisting of phenyl, naphthyl, indenyl, indanyl, tetralinyl, C₃₋₁₀ cycloalkyl, 3- to 10-membered heterocyclyl, and 8- to 11-membered heterobicyclyl; wherein each T⁰ is independently optionally substituted with one or more -R^{x2}, which are the same or different;
each -R^{x2} is independently selected from the group consisting of halogen, -CN, oxo (=O), -COOR^{x4}, -OR^{x4}, -C(O)R^{x4}, -C(O)N(R^{x4}R^{x4a}), -S(O)₂N(R^{x4}R^{x4a}), -S(O)N(R^{x4}R^{x4a}), -S(O)₂R^{x4}, -S(O)R^{x4}, -N(R^{x4})S(O)₂N(R^{x4a}R^{x4b}), -SR^{x4}, -N(R^{x4}R^{x4a}), -NO₂, -OC(O)R^{x4}, -N(R^{x4})C(O)R^{x4a}, -N(R^{x4})S(O)₂R^{x4a}, -N(R^{x4})S(O)R^{x4a}, -N(R^{x4})C(O)OR^{x4a}, -N(R^{x4})C(O)N(R^{x4a}R^{x4b}), -OC(O)N(R^{x4}R^{x4a}), and C₁₋₆ alkyl; wherein C₁₋₆ alkyl is optionally substituted with one or more halogen, which are the same or different;
each -R^{x3}, -R^{x3a}, -R^{x4}, -R^{x4a}, -R^{x4b} is independently selected from the group consisting of -H and C₁₋₆ alkyl; wherein C₁₋₆ alkyl is optionally substituted with one or more halogen, which are the same or different.

More preferably, the one or more further optional substituents are independently of each other selected from the group consisting of halogen, -CN, -COOR^{x1}, -OR^{x1}, -C(O)R^{x1}, -C(O)N(R^{x1}R^{x1a}), -S(O)₂N(R^{x1}R^{x1a}), -S(O)N(R^{x1}R^{x1a}), -S(O)₂R^{x1}, -S(O)R^{x1}, -N(R^{x1})S(O)₂N(R^{x1a}R^{x1b}), -SR^{x1}, -N(R^{x1}R^{x1a}), -NO₂, -OC(O)R^{x1}, -N(R^{x1})C(O)R^{x1a}, -N(R^{x1})S(O)₂R^{x1a}, -N(R^{x1})S(O)R^{x1a}, -N(R^{x1})C(O)OR^{x1a}, -N(R^{x1})C(O)N(R^{x1a}R^{x1b}), -OC(O)N(R^{x1}R^{x1a}), -T⁰, C₁₋₁₀ alkyl, C₂₋₁₀ alkenyl, and C₂₋₁₀ alkynyl; wherein -T⁰, C₁₋₁₀ alkyl, C₂₋₁₀ alkenyl, and C₂₋₁₀ alkynyl are optionally substituted with one or more -R^{x2}, which are the same or different and wherein C₁₋₁₀ alkyl, C₂₋₁₀ alkenyl, and C₂₋₁₀ alkynyl are optionally interrupted by one or more groups selected from the group consisting of -T⁰-, -C(O)O-, -O-, -C(O)-, -C(O)N(R^{x3})-, -S(O)₂N(R^{x3})-, -S(O)N(R^{x3})-, -S(O)₂-, -S(O)-, -N(R^{x3})S(O)₂N(R^{x3a})-, -S-, -N(R^{x3})-, -OC(OR^{x3})(R^{x3a})-, -N(R^{x3})C(O)N(R^{x3a})-, and -OC(O)N(R^{x3})-;
each -R^{x1}, -R^{x1a}, -R^{x1b}, -R^{x3}, -R^{x3a} is independently selected from the group consisting of -H, halogen, C₁₋₆ alkyl, C₂₋₆ alkenyl, and C₂₋₆ alkynyl;
each T⁰ is independently selected from the group consisting of phenyl, naphthyl, indenyl, indanyl, tetralinyl, C₃₋₁₀ cycloalkyl, 3- to 10-membered heterocyclyl, and 8- to 11-membered heterobicyclyl; wherein each T⁰ is independently optionally substituted with one or more -R^{x2}, which are the same or different;
each -R^{x2} is independently selected from the group consisting of halogen, -CN, oxo (=O), -COOR^{x4}, -OR^{x4}, -C(O)R^{x4}, -C(O)N(R^{x4}R^{x4a}), -S(O)₂N(R^{x4}R^{x4a}), -S(O)N(R^{x4}R^{x4a}), -S(O)₂R^{x4}, -S(O)R^{x4}, -N(R^{x4})S(O)₂N(R^{x4a}R^{x4b}), -SR^{x4}, -N(R^{x4}R^{x4a}), -NO₂, -OC(O)R^{x4}, -N(R^{x4})C(O)R^{x4a}, -N(R^{x4})S(O)₂R^{x4a}, -N(R^{x4})S(O)R^{x4a}, -N(R^{x4})C(O)OR^{x4a}, -N(R^{x4})C(O)N(R^{x4a}R^{x4b}), -OC(O)N(R^{x4}R^{x4a}), and C₁₋₆ alkyl; wherein C₁₋₆ alkyl is optionally substituted with one or more halogen, which are the same or different;
each -R^{x4}, -R^{x4a}, -R^{x4b} is independently selected from the group consisting of -H, halogen, C₁₋₆ alkyl, C₂₋₆ alkenyl, and C₂₋₆ alkynyl;
Even more preferably, the one or more further optional substituents are independently of each other selected from the group consisting of halogen, -CN, -COOR^{x1}, -OR^{x1}, -C(O)R^{x1}, -C(O)N(R^{x1}R^{x1a}), -S(O)₂N(R^{x1}R^{x1a}), -S(O)N(R^{x1}R^{x1a}), -S(O)₂R^{x1}, -S(O)R^{x1}, -N(R^{x1})S(O)₂N(R^{x1a}R^{x1b}), -SR^{x1}, -N(R^{x1}R^{x1a}), -NO₂, -OC(O)R^{x1}, -N(R^{x1})C(O)R^{x1a}, -N(R^{x1})S(O)₂R^{x1a}, -N(R^{x1})S(O)R^{x1a}, -N(R^{x1})C(O)OR^{x1a}, -N(R^{x1})C(O)N(R^{x1a}R^{x1b}), -OC(O)N(R^{x1}R^{x1a}), -T⁰, C₁₋₆ alkyl, C₂₋₆ alkenyl, and C₂₋₆ alkynyl; wherein -T⁰, C₁₋₆ alkyl, C₂₋₆ alkenyl, and C₂₋₆ alkynyl are optionally substituted with one or more -R^{x2}, which are the same or different and wherein C₁₋₆ alkyl, C₂₋₆ alkenyl, and C₂₋₆ alkynyl are optionally interrupted by one or more groups selected from the group consisting of -T⁰-, -C(O)O-, -O-, -C(O)-, -C(O)N(R^{x3})-, -S(O)₂N(R^{x3})-, -S(O)N(R^{x3})-, -S(O)₂-, -S(O)-, -N(R^{x3})S(O)₂N(R^{x3a})-, -S-, -N(R^{x3})-, -OC(OR^{x3})(R^{x3a})-, -N(R^{x3})C(O)N(R^{x3a})-, and -OC(O)N(R^{x3})-;
each -R^{x1}, -R^{x1a}, -R^{x1b}, -R^{x2}, -R^{x3}, -R^{x3a} is independently selected from the group consisting of -H, halogen, C₁₋₆ alkyl, C₂₋₆ alkenyl, and C₂₋₆ alkynyl;
each T⁰ is independently selected from the group consisting of phenyl, naphthyl, indenyl, indanyl, tetralinyl, C₃₋₁₀ cycloalkyl, 3- to 10-membered heterocyclyl, and 8- to 11-membered heterobicyclyl; wherein each T⁰ is independently optionally substituted with one or more -R^{x2}, which are the same or different.

Preferably, a maximum of 6 -H atoms of an optionally substituted molecule or moiety are independently replaced by a substituent, e.g. 5 -H atoms are independently replaced by a substituent, 4 -H atoms are independently replaced by a substituent, 3 -H atoms are independently replaced by a substituent, 2 -H atoms are independently replaced by a substituent, or 1 -H atom is replaced by a substituent.

The term "interrupted" means that a moiety is inserted between two carbon atoms or - if the insertion is at one of the moiety's ends - between a carbon or heteroatom and a hydrogen atom, preferably between a carbon and a hydrogen atom.

The term "spacer" refers to any moiety that is suitable to connect two moieties. Preferably, a spacer is selected from the group consisting of -T-, -C(O)O-, -O-, -C(O)-, -C(O)N(R^{y1})-, -S(O)₂N(R^{y1})-, -S(O)N(R^{y1})-, -S(O)₂-, -S(O)-, -N(R^{y1})S(O)₂N(R^{y1a})-, -S-, -N(R^{y1})-, -OC(OR^{y1})(R^{y1a})-, -N(R^{y1})C(O)N(R^{y1a})-, -OC(O)N(R^{y1})-, C₁₋₅₀ alkyl, C₂₋₅₀ alkenyl, and C₂₋₅₀ alkynyl; wherein -T-, C₁₋₅₀ alkyl, C₂₋₅₀ alkenyl, and C₂₋₅₀ alkynyl are optionally substituted with one or more -R^{y2}, which are the same or different and wherein C₁₋₅₀ alkyl, C₂₋₅₀ alkenyl, and C₂₋₅₀ alkynyl are optionally interrupted by one or more groups selected from the group consisting of -T-, -C(O)O-, -O-, -C(O)-, -C(O)N(R^{y3})-, -S(O)₂N(R^{y3})-, -S(O)N(R^{y3})-, -S(O)₂-, -S(O)-, -N(R^{y3})S(O)₂N(R^{y3a})-, -S-, -N(R^{y3})-, -OC(OR^{y3})(R^{y3a})-, -N(R^{y3})C(O)N(R^{y3a})-, and -OC(O)N(R^{y3})-;
-R^{y1} and -R^{y1a} are independently of each other selected from the group consisting of -H, -T, C₁₋₅₀ alkyl, C₂₋₅₀ alkenyl, and C₂₋₅₀ alkynyl; wherein -T, C₁₋₅₀ alkyl, C₂₋₅₀ alkenyl, and C₂₋₅₀ alkynyl are optionally substituted with one or more -R^{y2}, which are the same or different, and wherein C₁₋₅₀ alkyl, C₂₋₅₀ alkenyl, and C₂₋₅₀ alkynyl are optionally interrupted by one or more groups selected from the group consisting of -T-, -C(O)O-, -O-, -C(O)-, -C(O)N(R^{y4})-, -S(O)₂N(R^{y4})-, -S(O)N(R^{y4})-, -S(O)₂-, -S(O)-, -N(R^{y4})S(O)₂N(R^{y4a})-, -S-, -N(R^{y4})-, -OC(OR^{y4})(R^{y4a})-, -N(R^{y4})C(O)N(R^{y4a})-, and -OC(O)N(R^{y4})-;
each T is independently selected from the group consisting of phenyl, naphthyl, indenyl, indanyl, tetralinyl, C₃₋₁₀ cycloalkyl, 3- to 10-membered heterocyclyl, 8- to 11-membered heterobicyclyl, 8-to 30-membered carbopolycyclyl, and 8- to 30-membered heteropolycyclyl; wherein each T is independently optionally substituted with one or more -R^{y2}, which are the same or different;
each -R^{y2} is independently selected from the group consisting of halogen, -CN, oxo (=O), -COOR^{y5}, -OR^{y5}, -C(O)R^{y5}, -C(O)N(R^{y5}R^{y5a}), -S(O)₂N(R^{y5}R^{y5a}), -S(O)N(R^{y5}R^{y5a}), -S(O)₂R^{y5}, -S(O)R^{y5}, -N(R^{y5})S(O)₂N(R^{y5a}R^{y5b}), -SR^{y5}, -N(R^{y5}R^{y5a}), -NO₂, -OC(O)R^{y5}, -N(R^{y5})C(O)R^{y5a}, -N(R^{y5})S(O)₂R^{y5a}, -N(R^{y5})S(O)R^{y5a}, -N(R^{y5})C(O)OR^{y5a}, -N(R^{y5})C(O)N(R^{y5a}R^{y5b}), -OC(O)N(R^{y5}R^{y5a}), and C₁₋₆ alkyl; wherein C₁₋₆ alkyl is optionally substituted with one or more halogen, which are the same or different; and
each -R^{y3}, -R^{y3a}, -R^{y4}, -R^{y4a}, -R^{y5}, -R^{y5a} and -R^{y5b} is independently selected from the group consisting of -H, and C₁₋₆ alkyl, wherein C₁₋₆ alkyl is optionally substituted with one or more halogen, which are the same or different.

Even more preferably the spacer is selected from -T-, -C(O)O-, -O-, -C(O)-, -C(O)N(R^{y1})-, -S(O)₂N(R^{y1})-, -S(O)N(R^{y1})-, -S(O)₂-, -S(O)-, -N(R^{y1})S(O)₂N(R^{y1a})-, -S-, -N(R^{y1})-, -OC(OR^{y1})(R^{y1a})-, -N(R^{y1})C(O)N(R^{y1a})-, -OC(O)N(R^{y1})-, C₁₋₅₀ alkyl, C₂₋₅₀ alkenyl, and C₂₋₅₀ alkynyl; wherein -T-, C₁₋₂₀ alkyl, C₂₋₂₀ alkenyl, and C₂₋₂₀ alkynyl are optionally substituted with one or more -R^{y2}, which are the same or different and wherein C₁₋₂₀ alkyl, C₂₋₂₀ alkenyl, and C₂₋₂₀ alkynyl are optionally interrupted by one or more groups selected from the group consisting of -T-, -C(O)O-, -O-, -C(O)-, -C(O)N(R^{y3})-, -S(O)₂N(R^{y3})-, -S(O)N(R^{y3})-, -S(O)₂-, -S(O)-, -N(R^{y3})S(O)₂N(R^{y3a})-, -S-, -N(R^{y3})-, -OC(OR^{y3})(R^{y3a})-, -N(R^{y3})C(O)N(R^{y3a})-, and -OC(O)N(R^{y3})-;
-R^{y1} and -R^{y1a} are independently of each other selected from the group consisting of -H, -T, C₁₋₁₀ alkyl, C₂₋₁₀ alkenyl, and C₂₋₁₀ alkynyl; wherein -T, C₁₋₁₀ alkyl, C₂₋₁₀ alkenyl, and C₂₋₁₀ alkynyl are optionally substituted with one or more -R^{y2}, which are the same or different, and wherein C₁₋₁₀ alkyl, C₂₋₁₀ alkenyl, and C₂₋₁₀ alkynyl are optionally interrupted by one or more groups selected from the group consisting of -T-, -C(O)O-, -O-, -C(O)-, -C(O)N(R^{y4})-, -S(O)₂N(R^{y4})-, -S(O)N(R^{y4})-, -S(O)₂-, -S(O)-, -N(R^{y4})S(O)₂N(R^{y4a})-, -S-, -N(R^{y4})-, -OC(OR^{y4})(R^{y4a})-, -N(R^{y4})C(O)N(R^{y4a})-, and -OC(O)N(R^{y4})-;
each T is independently selected from the group consisting of phenyl, naphthyl, indenyl, indanyl, tetralinyl, C₃₋₁₀ cycloalkyl, 3- to 10-membered heterocyclyl, 8- to 11-membered heterobicyclyl, 8-to 30-membered carbopolycyclyl, and 8- to 30-membered heteropolycyclyl; wherein each T is independently optionally substituted with one or more -R^{y2}, which are the same or different;
-R^{y2} is selected from the group consisting of halogen, -CN, oxo (=O), -COOR^{y5}, -OR^{y5}, -C(O)R^{y5}, -C(O)N(R^{y5}R^{y5a}), -S(O)₂N(R^{y5}R^{y5a}), -S(O)N(R^{y5}R^{y5a}), -S(O)₂R^{y5}, -S(O)R^{y5}, -N(R^{y5})S(O)₂N(R^{y5a}R^{y5b}), -SR^{y5}, -N(R^{y5}R^{y5a}), -NO₂, -OC(O)R^{y5}, -N(R^{y5})C(O)R^{y5a}, -N(R^{y5})S(O)₂R^{y5a}, -N(R^{y5})S(O)R^{y5a}, -N(R^{y5})C(O)OR^{y5a}, -N(R^{y5})C(O)N(R^{y5a}R^{y5b}), -OC(O)N(R^{y5}R^{y5a}), and C₁₋₆ alkyl; wherein C₁₋₆ alkyl is optionally substituted with one or more halogen, which are the same or different; and
each -R^{y3}, -R^{y3a}, -R^{y4}, -R^{y4a}, -R^{y5}, -R^{y5a} and -R^{y5b} is independently of each other selected from the group consisting of -H, and C₁₋₆ alkyl; wherein C₁₋₆ alkyl is optionally substituted with one or more halogen, which are the same or different.

Even more preferably the spacer is selected from the group consisting of -T-, -C(O)O-, -O-, -C(O)-, -C(O)N(R^{y1})-, -S(O)₂N(R^{y1})-, -S(O)N(R^{y1})-, -S(O)₂-, -S(O)-, -N(R^{y1})S(O)₂N(R^{y1a})-, -S-, -N(R^{y1})-, -OC(OR^{y1})(R^{y1a})-, -N(R^{y1})C(O)N(R^{y1a})-, -OC(O)N(R^{y1})-, C₁₋₅₀ alkyl, C₂₋₅₀ alkenyl, and C₂₋₅₀ alkynyl; wherein -T-, C₁₋₅₀ alkyl, C₂₋₅₀ alkenyl, and C₂₋₅₀ alkynyl are optionally substituted with one or more -R^{y2}, which are the same or different and wherein C₁₋₅₀ alkyl, C₂₋₅₀ alkenyl, and C₂₋₅₀ alkynyl are optionally interrupted by one or more groups selected from the group consisting of -T-, -C(O)O-, -O-, -C(O)-, -C(O)N(R^{y3})-, -S(O)₂N(R^{y3})-, -S(O)N(R^{y3})-, -S(O)₂-, -S(O)-, -N(R^{y3})S(O)₂N(R^{y3a})-, -S-, -N(R^{y3})-, -OC(OR^{y3})(R^{y3a})-, -N(R^{y3})C(O)N(R^{y3a})-, and -OC(O)N(R^{y3})-;
-R^{y1} and -R^{y1a} are independently selected from the group consisting of -H, -T, C₁₋₁₀ alkyl, C₂₋₁₀ alkenyl, and C₂₋₁₀ alkynyl;
each T is independently selected from the group consisting of phenyl, naphthyl, indenyl, indanyl, tetralinyl, C₃₋₁₀ cycloalkyl, 3- to 10-membered heterocyclyl, 8- to 11-membered heterobicyclyl, 8-to 30-membered carbopolycyclyl, and 8- to 30-membered heteropolycyclyl;
each -R^{y2} is independently selected from the group consisting of halogen, and C₁₋₆ alkyl; and
each -R^{y3}, -R^{y3a}, -R^{y4}, -R^{y4a}, -R^{y5}, -R^{y5a} and -R^{y5b} is independently of each other selected from the group consisting of -H, and C₁₋₆ alkyl; wherein C₁₋₆ alkyl is optionally substituted with one or more halogen, which are the same or different.

As used herein, the term "C₁₋₄ alkyl" alone or in combination means a straight-chain or branched alkyl moiety having 1 to 4 carbon atoms. If present at the end of a molecule, examples of straight-chain or branched C₁₋₄ alkyl are methyl, ethyl, n-propyl, isopropyl, n-butyl, isobutyl, sec-butyl and tert-butyl. When two moieties of a molecule are linked by the C₁₋₄ alkyl, then examples for such C₁₋₄ alkyl groups are -CH₂-, -CH₂-CH₂-, -CH(CH₃)-, -CH₂-CH₂-CH₂-, -CH(C₂H₅)-, -C(CH₃)₂-. Each hydrogen of a C₁₋₄ alkyl carbon may optionally be replaced by a substituent as defined above. Optionally, a C₁₋₄ alkyl may be interrupted by one or more moieties as defined below.

As used herein, the term "C₁₋₆ alkyl" alone or in combination means a straight-chain or branched alkyl moiety having 1 to 6 carbon atoms. If present at the end of a molecule, examples of straight-chain and branched C₁₋₆ alkyl groups are methyl, ethyl, n-propyl, isopropyl, n-butyl, isobutyl, sec-butyl, tert-butyl, n-pentyl, 2-methylbutyl, 2,2-dimethylpropyl, n-hexyl, 2-methylpentyl, 3-methylpentyl, 2,2-dimethylbutyl, 2,3-dimethylbutyl and 3,3-dimethylpropyl. When two moieties of a molecule are linked by the C₁₋₆ alkyl group, then examples for such C₁₋₆ alkyl groups are -CH₂-, -CH₂-CH₂-, -CH(CH₃)-, -CH₂-CH₂-CH₂-, -CH(C₂H₅)- and -C(CH₃)₂-. Each hydrogen atom of a C₁₋₆ carbon may optionally be replaced by a substituent as defined above. Optionally, a C₁₋₆ alkyl may be interrupted by one or more moieties as defined below.

Accordingly, "C₁₋₁₀ alkyl", "C₁₋₂₀ alkyl" or "C₁₋₅₀ alkyl" means an alkyl chain having 1 to 10, 1 to 20 or 1 to 50 carbon atoms, respectively, wherein each hydrogen atom of the C₁₋₁₀, C₁₋₂₀ or C₁₋₅₀ carbon may optionally be replaced by a substituent as defined above. Optionally, a C₁₋₁₀ or C₁₋₅₀ alkyl may be interrupted by one or more moieties as defined below.

As used herein, the term "C₂₋₆ alkenyl" alone or in combination means a straight-chain or branched hydrocarbon moiety comprising at least one carbon-carbon double bond having 2 to 6 carbon atoms. If present at the end of a molecule, examples are -CH=CH₂, -CH=CH-CH₃, -CH₂-CH=CH₂, -CH=CHCH₂-CH₃ and -CH=CH-CH=CH₂. When two moieties of a molecule are linked by the C₂₋₆ alkenyl group, then an example for such C₂₋₆ alkenyl is -CH=CH-. Each hydrogen atom of a C₂₋₆ alkenyl moiety may optionally be replaced by a substituent as defined above. Optionally, a C₂₋₆ alkenyl may be interrupted by one or more moieties as defined below.

Accordingly, the term "C₂₋₁₀ alkenyl", "C₂₋₂₀ alkenyl" or "C₂₋₅₀ alkenyl" alone or in combination means a straight-chain or branched hydrocarbon moiety comprising at least one carbon-carbon double bond having 2 to 10, 2 to 20 or 2 to 50 carbon atoms. Each hydrogen atom of a C₂₋₁₀ alkenyl, C₂₋₂₀ alkenyl or C₂₋₅₀ alkenyl group may optionally be replaced by a substituent as defined above. Optionally, a C₂₋₁₀ alkenyl, C₂₋₂₀ alkenyl or C₂₋₅₀ alkenyl may be interrupted by one or more moieties as defined below.

As used herein, the term "C₂₋₆ alkynyl" alone or in combination means straight-chain or branched hydrocarbon moiety comprising at least one carbon-carbon triple bond having 2 to 6 carbon atoms. If present at the end of a molecule, examples are -C≡CH, -CH₂-C≡CH, CH₂-CH₂-C≡CH and CH₂-C≡C-CH₃. When two moieties of a molecule are linked by the alkynyl group, then an example is -C≡C-. Each hydrogen atom of a C₂₋₆ alkynyl group may optionally be replaced by a substituent as defined above. Optionally, one or more double bond(s) may occur. Optionally, a C₂₋₆ alkynyl may be interrupted by one or more moieties as defined below.

Accordingly, as used herein, the term "C₂₋₁₀ alkynyl", "C₂₋₂₀ alkynyl" and "C₂₋₅₀ alkynyl" alone or in combination means a straight-chain or branched hydrocarbon moiety comprising at least one carbon-carbon triple bond having 2 to 10, 2 to 20 or 2 to 50 carbon atoms, respectively. Each hydrogen atom of a C₂₋₁₀ alkynyl, C₂₋₂₀ alkynyl or C₂₋₅₀ alkynyl group may optionally be replaced by a substituent as defined above. Optionally, one or more double bond(s) may occur. Optionally, a C₂₋₁₀ alkynyl, C₂₋₂₀ alkynyl or C₂₋₅₀ alkynyl may be interrupted by one or more moieties as defined below.

As mentioned above, a C₁₋₄ alkyl, C₁₋₆ alkyl, C₁₋₁₀ alkyl, C₁₋₂₀ alkyl, C₁₋₅₀ alkyl, C₂₋₆ alkenyl, C₂₋₁₀ alkenyl, C₂₋₂₀ alkenyl, C₂₋₅₀ alkenyl, C₂₋₆ alkynyl, C₂₋₁₀ alkynyl, C₂₋₂₀ alkenyl or C₂₋₅₀ alkynyl may optionally be interrupted by one or more moieties, which are preferably selected from the group consisting of 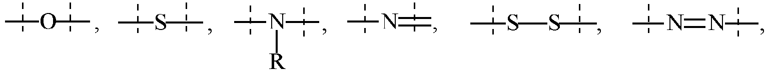 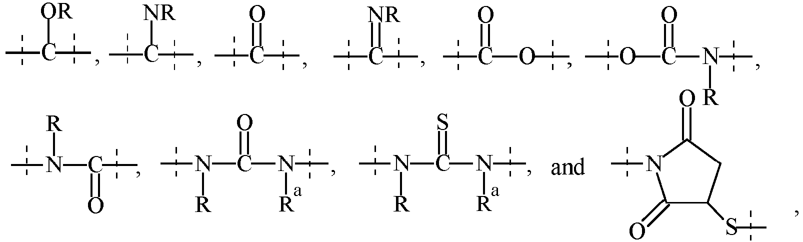 wherein
dashed lines indicate attachment to the remainder of the moiety or reagent; and
-R and -R^{a} are independently of each other selected from the group consisting of -H, methyl, ethyl, propyl, butyl, pentyl and hexyl.

As used herein, the term "C₃₋₁₀ cycloalkyl" means a cyclic alkyl chain having 3 to 10 carbon atoms, which may be saturated or unsaturated, e.g. cyclopropyl, cyclobutyl, cyclopentyl, cyclohexyl, cyclohexenyl, cycloheptyl, cyclooctyl, cyclononyl or cyclodecyl. Each hydrogen atom of a C₃₋₁₀ cycloalkyl carbon may be replaced by a substituent as defined above. The term "C₃₋₁₀ cycloalkyl" also includes bridged bicycles like norbornane or norbornene.

The term "8- to 30-membered carbopolycyclyl" or "8- to 30-membered carbopolycycle" means a cyclic moiety of two or more rings with 8 to 30 ring atoms, where two neighboring rings share at least one ring atom and that may contain up to the maximum number of double bonds (aromatic or non-aromatic ring which is fully, partially or un-saturated). Preferably a 8- to 30-membered carbopolycyclyl means a cyclic moiety of two, three, four or five rings, more preferably of two, three or four rings.

As used herein, the term "3- to 10-membered heterocyclyl" or "3- to 10-membered heterocycle" means a ring with 3, 4, 5, 6, 7, 8, 9 or 10 ring atoms that may contain up to the maximum number of double bonds (aromatic or non-aromatic ring which is fully, partially or un-saturated) wherein at least one ring atom up to 4 ring atoms are replaced by a heteroatom selected from the group consisting of sulfur (including -S(O)-, -S(O)₂-), oxygen and nitrogen (including =N(O)-) and wherein the ring is linked to the rest of the molecule via a carbon or nitrogen atom. Examples for 3- to 10-membered heterocycles include but are not limited to aziridine, oxirane, thiirane, azirine, oxirene, thiirene, azetidine, oxetane, thietane, furan, thiophene, pyrrole, pyrroline, imidazole, imidazoline, pyrazole, pyrazoline, oxazole, oxazoline, isoxazole, isoxazoline, thiazole, thiazoline, isothiazole, isothiazoline, thiadiazole, thiadiazoline, tetrahydrofuran, tetrahydrothiophene, pyrrolidine, imidazolidine, pyrazolidine, oxazolidine, isoxazolidine, thiazolidine, isothiazolidine, thiadiazolidine, sulfolane, pyran, dihydropyran, tetrahydropyran, imidazolidine, pyridine, pyridazine, pyrazine, pyrimidine, piperazine, piperidine, morpholine, tetrazole, triazole, triazolidine, tetrazolidine, diazepane, azepine and homopiperazine. Each hydrogen atom of a 3- to 10-membered heterocyclyl or 3-to 10-membered heterocyclic group may be replaced by a substituent as defined below.

As used herein, the term "8- to 11-membered heterobicyclyl" or "8- to 11-membered heterobicycle" means a heterocyclic moiety of two rings with 8 to 11 ring atoms, where at least one ring atom is shared by both rings and that may contain up to the maximum number of double bonds (aromatic or non-aromatic ring which is fully, partially or un-saturated) wherein at least one ring atom up to 6 ring atoms are replaced by a heteroatom selected from the group consisting of sulfur (including -S(O)-, -S(O)₂-), oxygen and nitrogen (including =N(O)-) and wherein the ring is linked to the rest of the molecule via a carbon or nitrogen atom. Examples for an 8- to 11-membered heterobicycle are indole, indoline, benzofuran, benzothiophene, benzoxazole, benzisoxazole, benzothiazole, benzisothiazole, benzimidazole, benzimidazoline, quinoline, quinazoline, dihydroquinazoline, quinoline, dihydroquinoline, tetrahydroquinoline, decahydroquinoline, isoquinoline, decahydroisoquinoline, tetrahydroisoquinoline, dihydroisoquinoline, benzazepine, purine and pteridine. The term 8- to 11-membered heterobicycle also includes spiro structures of two rings like 1,4-dioxa-8-azaspiro[4.5]decane or bridged heterocycles like 8-aza-bicyclo[3.2.1]octane. Each hydrogen atom of an 8- to 11-membered heterobicyclyl or 8- to 11-membered heterobicycle carbon may be replaced by a substituent as defined below.

Similary, the term "8- to 30-membered heteropolycyclyl" or "8- to 30-membered heteropolycycle" means a heterocyclic moiety of more than two rings with 8 to 30 ring atoms, preferably of three, four or five rings, where two neighboring rings share at least one ring atom and that may contain up to the maximum number of double bonds (aromatic or non-aromatic ring which is fully, partially or unsaturated), wherein at least one ring atom up to 10 ring atoms are replaced by a heteroatom selected from the group of sulfur (including -S(O)-, -S(O)₂-), oxygen and nitrogen (including =N(O)-) and wherein the ring is linked to the rest of a molecule via a carbon or nitrogen atom.

It is understood that the phrase "the pair R^{x}/R^{y} is joined together with the atom to which they are attached to form a C₃₋₁₀ cycloalkyl or a 3- to 10-membered heterocyclyl" in relation with a moiety of the structure 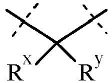 means that Rx and Ry form the following structure: 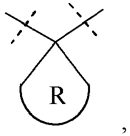 wherein R is C₃₋₁₀ cycloalkyl or 3- to 10-membered heterocyclyl.

It is also understood that the phrase "the pair R^{x}/R^{y} is joint together with the atoms to which they are attached to form a ring A" in relation with a moiety of the structure 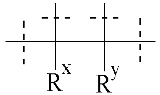 means that R^{x} and R^{y} form the following structure:

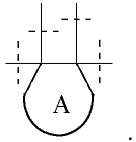

As used herein, "halogen" means fluoro, chloro, bromo or iodo. It is generally preferred that halogen is fluoro or chloro.

In general, the term "comprise" or "comprising" also encompasses "consist of" or "consisting of'.

The starting dose ranges from 0.8 to 4.9 nmol/day, preferably from 1 to 4.8 nmol/day, more preferably from 1.2 to 4.7 nmol/day, even more preferably from 1.5 to 4.6 nmol/day, even more preferably from 1.8 to 4.5 nmol/day and most preferably from 2 to 4.4 nmol/day.

In certain embodiments the starting dose ranges from 0.8 to 3.9 nmol/day, preferably from 1 to 3.7 nmol/day, even more preferably from 1.5 to 3.4 nmol/day, even more preferably from 2 to 3.2 nmol/day and most preferably from 2.5 to 3 nmol/day.

In certain embodiments the starting dose is at least 0.8 nmol/day. In certain embodiments the starting dose is at least 1.0 nmol/day. In certain embodiments the starting dose is at least 1.2 nmol/day. In certain embodiments the starting dose is at least 1.4 nmol/day. In certain embodiments the starting dose is at least 1.6 nmol/day. In certain embodiments the starting dose is at least 1.8 nmol/day. In certain embodiments the starting dose is at least 1.9 nmol/day. In certain embodiments the starting dose is at least 2.0 nmol/day. In certain embodiments the starting dose is at least 2.1 nmol/day. In certain embodiments the starting dose is at least 2.2 nmol/day. In certain embodiments the starting dose is at least 2.3 nmol/day. In certain embodiments the starting dose is at least 2.4 nmol/day. In certain embodiments the starting dose is at least 2.5 nmol/day. In certain embodiments the starting dose is at least 2.6 nmol/day. In certain embodiments the starting dose is at least 2.7 nmol/day. In certain embodiments the starting dose is at least 2.8 nmol/day. In certain embodiments the starting dose is at least 2.9 nmol/day. In certain embodiments the starting dose is at least 3.0 nmol/day. In certain embodiments the starting dose is at least 3.1 nmol/day. In certain embodiments the starting dose is at least 3.2 nmol/day. In certain embodiments the starting dose is at least 3.3 nmol/day. In certain embodiments the starting dose is at least 3.4 nmol/day. In certain embodiments the starting dose is at least 3.5 nmol/day. In certain embodiments the starting dose is at least 3.6 nmol/day. In certain embodiments the starting dose is at least 3.7 nmol/day. In certain embodiments the starting dose is at least 3.8 nmol/day. In certain embodiments the starting dose is at least 3.9 nmol/day. In certain embodiments the starting dose is at least 4.0 nmol/day.

The starting dose is at most 4.9 nmol/day. In certain embodiments the starting dose is at most 4.8 nmol/day. In certain embodiments the starting dose is at most 4.7 nmol/day. In certain embodiments the starting dose is at most 4.6 nmol/day. In certain embodiments the starting dose is at most 4.5 nmol/day. In certain embodiments the starting dose is at most 4.4 nmol/day. In certain embodiments the starting dose is at most 4.3 nmol/day. In certain embodiments the starting dose is at most 4.2 nmol/day. In certain embodiments the starting dose is at most 4.1 nmol/day. In certain embodiments the starting dose is at most 4.0 nmol/day. In certain embodiments the starting dose is at most 3.9 nmol/day.

In certain embodiments the starting dose is at least 2.9 nmol/day and at most 4.9 nmol/day. In certain embodiments the starting dose ist at least 2.9 nmol/day and at most 4.5 nmol/day.

In one embodiment the starting dose is 1.5 ± 0.3 nmol/day, preferably 1.5 ± 0.2 nmol/day, even more preferably 1.5 ± 0.1 nmol/day. In another embodiment the starting dose is 1.7 ± 0.3 nmol/day, preferably 1.7 ± 0.2 nmol/day, even more preferably 1.7 ± 0.1 nmol/day. In another embodiment the starting dose is 2 ± 0.3 nmol/day, preferably 2 ± 0.2 nmol/day, even more preferably 2 ± 0.1 nmol/day. In another embodiment the starting dose is 2.3 ± 0.3 nmol/day, preferably 2.3 ± 0.2 nmol/day, even more preferably 2.3 ± 0.1 nmol/day. In another embodiment the starting dose is 2.5 ± 0.3 nmol/day, preferably 2.5 ± 0.2 nmol/day, even more preferably 2.5 ± 0.1 nmol/day. In another embodiment the starting dose is 2.7 ± 0.3 nmol/day, preferably 2.7 ± 0.2 nmol/day, even more preferably 2.7 ± 0.1 nmol/day. In another embodiment the starting dose is 2.9 ± 0.3 nmol/day, preferably 2.9 ± 0.2 nmol/day, even more preferably 2.9 ± 0.1 nmol/day. In another embodiment the starting dose is 3.2 ± 0.3 nmol/day, preferably 3.2 ± 0.2 nmol/day, even more preferably 3.2 ± 0.1 nmol/day. In another embodiment the starting dose is 3.5 ± 0.3 nmol/day, preferably 3.5 ± 0.2 nmol/day, even more preferably 3.5 ± 0.1 nmol/day. In another embodiment the starting dose is 3.7 ± 0.3 nmol/day, preferably 3.7 ± 0.2 nmol/day, even more preferably 3.7 ± 0.1 nmol/day. In another embodiment the starting dose is 3.8 ± 0.3 nmol/day, preferably 3.8 ± 0.2 nmol/day, even more preferably 3.8 ± 0.1 nmol/day. In another embodiment the starting dose is 3.9 ± 0.3 nmol/day, preferably 3.9 ± 0.2 nmol/day, even more preferably 3.9 ± 0.1 nmol/day. In another embodiment the starting dose is 4.0 ± 0.3 nmol/day, preferably 4.0 ± 0.2 nmol/day, even more preferably 4.0 ± 0.1 nmol/day. In another embodiment the starting dose is 4.1 ± 0.3 nmol/day, preferably 4.1 ± 0.2 nmol/day, even more preferably 4.1 ± 0.1 nmol/day. In another embodiment the starting dose is 4.2 ± 0.3 nmol/day, preferably 4.2 ± 0.2 nmol/day, even more preferably 4.2 ± 0.1 nmol/day. In another embodiment the starting dose is 4.2 ± 0.3 nmol/day, preferably 4.2 ± 0.2 nmol/day, even more preferably 4.2 ± 0.1 nmol/day. In another embodiment the starting dose is 4.3 ± 0.3 nmol/day, preferably 4.3 ± 0.2 nmol/day, even more preferably 4.3 ± 0.1 nmol/day. In another embodiment the starting dose is 4.4 ± 0.3 nmol/day, preferably 4.4 ± 0.2 nmol/day, even more preferably 4.4 ± 0.1 nmol/day. In another embodiment the starting dose is 4.5 ± 0.3 nmol/day, preferably 4.5 ± 0.2 nmol/day, even more preferably 4.5 ± 0.1 nmol/day. In another embodiment the starting dose is 4.6 ± 0.3 nmol/day, preferably 4.6 ± 0.2 nmol/day, even more preferably 4.6 ± 0.1 nmol/day.

The disease that is treated with the PTH conjugate is hypoparathyroidism.

The patient suffering from hypoparathyroidism is a human patient.

Preferably the PTH conjugate or a pharmaceutically acceptable salt thereof or the pharmaceutical composition comprising said PTH conjugate or pharmaceutically acceptable salt thereof is administered to the patient daily, i.e. once every day. Even more preferably the PTH conjugate or a pharmaceutically acceptable salt thereof or the pharmaceutical composition comprising said PTH conjugate or pharmaceutically acceptable salt thereof is administered to the patient once every 24 hours.

According to the present invention, and claimed, the PTH conjugate of the present invention is of formula (IIf-i): 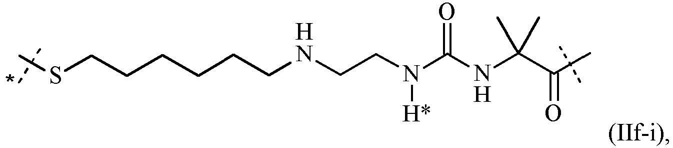 wherein
the unmarked dashed line indicates the attachment to a nitrogen of -D which is a PTH moiety by forming an amide bond; and
the dashed line marked with the asterisk indicates attachment to a moiety 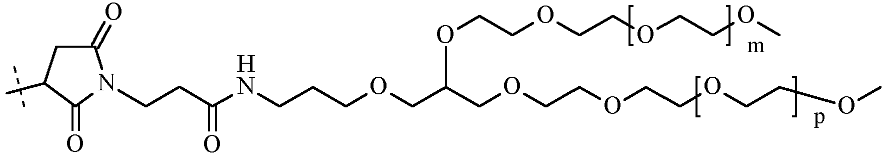
wherein
m and p are independently an integer ranging from and including 400 to 500.

-D is attached to the PTH conjugate of formula (IIf-i) through the N-terminal amine functional group of the PTH moiety.

If the PTH conjugate is present in a pharmaceutical composition such pharmaceutical composition comprises at least one PTH conjugate of the present invention and at least one excipient.

In one embodiment the pharmaceutical composition is a dry formulation. It is understood that such dry formulation requires reconstitution prior to administration to a patient. In another embodiment the pharmaceutical composition is a liquid formualation.

Such liquid or dry pharmaceutical composition comprises at least one excipient. Excipients used in parenteral formulations may be categorized as, for example, buffering agents, isotonicity modifiers, preservatives, stabilizers, anti-adsorption agents, oxidation protection agents, viscosifiers/viscosity enhancing agents, or other auxiliary agents. However, in some cases, one excipient may have dual or triple functions. Preferably, the at least one excipient comprised in the pharmaceutical composition of the present invention is selected from the group consisting of
(i) Buffering agents: physiologically tolerated buffers to maintain pH in a desired range, such as sodium phosphate, bicarbonate, succinate, histidine, citrate and acetate, sulphate, nitrate, chloride, pyruvate; antacids such as Mg(OH)₂ or ZnCO₃ may be also used;
(ii) Isotonicity modifiers: to minimize pain that can result from cell damage due to osmotic pressure differences at the injection depot; glycerin and sodium chloride are examples; effective concentrations can be determined by osmometry using an assumed osmolality of 285-315 mOsmol/kg for serum;
(iii) Preservatives and/or antimicrobials: multidose parenteral formulations require the addition of preservatives at a sufficient concentration to minimize risk of patients becoming infected upon injection and corresponding regulatory requirements have been established; typical preservatives include m-cresol, phenol, methylparaben, ethylparaben, propylparaben, butylparaben, chlorobutanol, benzyl alcohol, phenylmercuric nitrate, thimerosol, sorbic acid, potassium sorbate, benzoic acid, chlorocresol, and benzalkonium chloride;
(iv) Stabilizers: Stabilisation is achieved by strengthening of the protein-stabilising forces, by destabilisation of the denatured state, or by direct binding of excipients to the protein; stabilizers may be amino acids such as alanine, arginine, aspartic acid, glycine, histidine, lysine, proline, sugars such as glucose, sucrose, trehalose, polyols such as glycerol, mannitol, sorbitol, salts such as potassium phosphate, sodium sulphate, chelating agents such as EDTA, hexaphosphate, ligands such as divalent metal ions (zinc, calcium, etc.), other salts or organic molecules such as phenolic derivatives; in addition, oligomers or polymers such as cyclodextrins, dextran, dendrimers, PEG or PVP or protamine or HSA may be used;
(v) Anti-adsorption agents: Mainly ionic or non-ionic surfactants or other proteins or soluble polymers are used to coat or adsorb competitively to the inner surface of the formulation's container; e.g., poloxamer (Pluronic F-68), PEG dodecyl ether (Brij 35), polysorbate 20 and 80, dextran, polyethylene glycol, PEG-polyhistidine, BSA and HSA and gelatins; chosen concentration and type of excipient depends on the effect to be avoided but typically a monolayer of surfactant is formed at the interface just above the CMC value;
(vi) Oxidation protection agents: antioxidants such as ascorbic acid, ectoine, methionine, glutathione, monothioglycerol, morin, polyethylenimine (PEI), propyl gallate, and vitamin E; chelating agents such as citric acid, EDTA, hexaphosphate, and thioglycolic acid may also be used;
(vii) Viscosifiers or viscosity enhancers: in case of a suspension retard settling of the particles in the vial and syringe and are used in order to facilitate mixing and resuspension of the particles and to make the suspension easier to inject (i.e., low force on the syringe plunger); suitable viscosifiers or viscosity enhancers are, for example, carbomer viscosifiers like Carbopol 940, Carbopol Ultrez 10, cellulose derivatives like hydroxypropylmethylcellulose (hypromellose, HPMC) or diethylaminoethyl cellulose (DEAE or DEAE-C), colloidal magnesium silicate (Veegum) or sodium silicate, hydroxyapatite gel, tricalcium phosphate gel, xanthans, carrageenans like Satia gum UTC 30, aliphatic poly(hydroxy acids), such as poly(D,L- or L-lactic acid) (PLA) and poly(glycolic acid) (PGA) and their copolymers (PLGA), terpolymers of D,L-lactide, glycolide and caprolactone, poloxamers, hydrophilic poly(oxyethylene) blocks and hydrophobic poly(oxypropylene) blocks to make up a triblock of poly(oxyethylene)-poly(oxypropylene)-poly(oxyethylene) (e.g. Pluronic^{®}), polyetherester copolymer, such as a polyethylene glycol terephthalate/polybutylene terephthalate copolymer, sucrose acetate isobutyrate (SAIB), dextran or derivatives thereof, combinations of dextrans and PEG, polydimethylsiloxane, collagen, chitosan, polyvinyl alcohol (PVA) and derivatives, polyalkylimides, poly (acrylamide-co-diallyldimethyl ammonium (DADMA)), polyvinylpyrrolidone (PVP), glycosaminoglycans (GAGs) such as dermatan sulfate, chondroitin sulfate, keratan sulfate, heparin, heparan sulfate, hyaluronan, ABA triblock or AB block copolymers composed of hydrophobic A-blocks, such as polylactide (PLA) or poly(lactide-co-glycolide) (PLGA), and hydrophilic B-blocks, such as polyethylene glycol (PEG) or polyvinyl pyrrolidone; such block copolymers as well as the abovementioned poloxamers may exhibit reverse thermal gelation behavior (fluid state at room temperature to facilitate administration and gel state above sol-gel transition temperature at body temperature after injection);
(viii) Spreading or diffusing agent: modifies the permeability of connective tissue through the hydrolysis of components of the extracellular matrix in the intrastitial space such as hyaluronic acid, a polysaccharide found in the intercellular space of connective tissue; a spreading agent such as hyaluronidase temporarily decreases the viscosity of the extracellular matrix and promotes diffusion of injected drugs; and
(ix) Other auxiliary agents: such as wetting agents, viscosity modifiers, antibiotics, hyaluronidase; acids and bases such as hydrochloric acid and sodium hydroxide are auxiliary agents necessary for pH adjustment during manufacture.

The pharmaceutical composition comprising at least one PTH conjugate may be administered to a patient by various modes of administration, such as via topical, enteral or parenteral administration and by methods of external application, injection or infusion, including intraarticular, periarticular, intradermal, subcutaneous, intramuscular, intravenous, intraosseous, intraperitoneal, intrathecal, intracapsular, intraorbital, intravitreal, intratympanic, intravesical, intracardiac, transtracheal, subcuticular, subcapsular, subarachnoid, intraspinal, intraventricular, intrasternal injection and infusion, direct delivery to the brain via implanted device allowing delivery of the invention or the like to brain tissue or brain fluids (e.g., Ommaya Reservoir), direct intracerebroventricular injection or infusion, injection or infusion into brain or brain associated regions, injection into the subchoroidal space, retro-orbital injection and ocular instillation. Preferably the pharmaceutical composition comprising at least one PTH prodrug is administered via subcutaneous injection.

Preferably, the entire starting dose is administered to the patient in one step, preferably as one subcutaneous injection.

Subcutaneous injection is preferably done with a syringe and needle or with a pen injector, even more preferably with a pen injector.

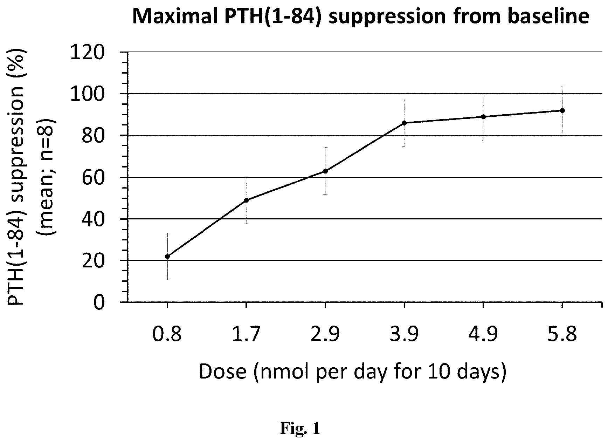

Figure 1 shows the mean of maximal PTH (1-84) suppression plotted for each dose level tested.

### Material and Methods

Compound 1 is shown below may be synthesized as described for compound 18 of example 18 of WO2017/148883A1:

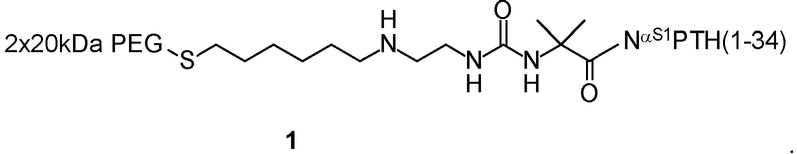

### Examples

### Example 1

To identify a safe starting dose for treating patients suffering from hypoparathyroidism, we investigated the pharmacodynamic effects of single and repeated daily dosing of a long-acting PTH molecule.

The safety, efficacy and pharmacokinetics of compound 1 was tested in a phase 1 study in healthy volunteers. The study investigated effects after single and multiple ascending doses in a randomized and placebo-controlled trial design. Each cohort consisted of 10 subjects (8 active, 2 placebo) and was administered either a single dose or 10 daily doses of TransCon PTH. The single dose cohorts were administered a single dose in the range 0.8-30.1 nmol while the multiple dose cohorts received one dose per day for 10 days in the range 0.8-5.8 nmol.

Compound 1 was generally well tolerated, with no drug-related serious or severe adverse events. The primary efficacy endpoints included albumin-adjusted serum calcium and endogenous secreted intact PTH(1-84).

The maximum tolerated dose (MTD) was 4.9 nmol/day of compound 1; 5 of 8 multiple ascending dose cohort participants (all female) who received compound 1 at 5.8, and both female placebo subjects, nmol/day had orthostatic hypotension, palpitations, and/or tachycardia and 1 had syncope. Two of 3 males had asymptomatic hypercalcemia. Observed adverse effects leading to MTD reflected known PTH pharmacology.

Total calcium was measured in serum by complexation using 2,2'-[1,8-Dihydroxy-3,6-disulphonaphthylene-2,7-bisazo]-bisbenzenearsonic acid (Arsenazo III, Beckman Coulter, Inc., USA) and detection by bichromatic absorbance at 600/700 nM. Albumin was measured by dye-binding method using 5,5-dibromo-o-cresolsulfonphthalein (Bromocresol Purple, Randox Laboratories, UK) and detection by bichromatic absorbance at 600/700 nm. Albumin-adjusted serum calcium was calculated as total calcium (mg/dL) + 0.8 (4 - albumin in g/dL). Intact PTH(1-84) was measured with the "Architect Intact PTH" assay from Abbott Laboratories; an in vitro chemiluminescent microparticle immunoassay (CMIA) for the quantitative determination of intact parathyroid hormone (PTH) in human serum and plasma. All analytical methods were basically performed as described by the manufacturer.

In the multiple dose cohorts albumin adjusted serum calcium was measured daily for two days before first dosing, before first dose and daily for two weeks after the first dose. Intact PTH(1-84) was measured the day before dosing, before first dose and 1, 2, 3, 4, 9, 11 and 13 days after the first dose.

The effect of compound 1 was demonstrated by dose dependent responses in the efficacy parameters as presented in figure 1 and the table below.

Maximal PTH (1-84) suppresion was calculated for individual subjects as the ratio (in percent) of the highest change from baseline during the sampling period and the difference between the subject's baseline and the assay's lower limit of quantification (3 pg/mL). Baseline was defined as the PTH(1-84) measurement before the first dose. The mean of maximal PTH (1-84) suppression was then plotted for each dose level (figure 1). Daily doses ranging from 0.8 to 3.9 nmol induced a linear suppression of PTH(1-84) secretion, with endogenous PTH(1-84) secretion reaching maximal suppression at doses above 3.9 nmol.

Maximal increase from baseline in albumin adjusted serum calcium was calculated for individual subjects as the highest change from baseline during that subject's sampling period. Baseline was calculated as the mean (n=3) of samples collected before dosing. The mean of maximal increase was calculated for each dose level and presented in the table below.

| **Dose level** | **Albumin adjusted Serum Calcium (mg/dL)** |
|---|---|
| (daily for 10 days) | Mean of maximal increase from baseline (n=8) |
| 0.8 nmol | 0.44 |
| 1.7 nmol | 0.35 |
| 2.9 nmol | 0.66 |
| 3.9 nmol | 0.82 |
| 4.9 nmol | 1.21 |
| 5.8 nmol | 1.42 |

It was surprisingly found that a starting dose for hypoparathyroidism patients for a PTH conjugate could be identified by gradually increasing the dose in healthy subjects. The range for the starting dose will be the lowest dose to increase serum calcium by about 0.3-0.4 mg/dL from baseline in healthy subjects, and upper dose range will be the highest dose that suppress endogenous PTH(1-84) secretion and does not cause hypercalcemia in healthy subjects.

### Abbreviations

- HP: hypoparathyroidism
- PTH: parathyroid hormone

## Claims

1. A PTH conjugate, in which a PTH moiety is reversibly conjugated to a polymeric moiety or a pharmaceutically acceptable salt thereof or a pharmaceutical composition comprising said PTH conjugate or pharmaceutically acceptable salt thereof for use in the treatment of hypoparathyroidism in a human patient, wherein the starting dose ranges from 0.8 to 4.9 nmol/day and wherein the PTH conjugate is of formula (IIf-i): 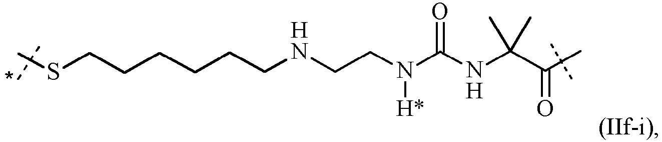 wherein
the unmarked dashed line indicates the attachment to the N-terminal amine functional group of a PTH moiety having the sequence of SEQ ID NO:51 by forming an amide bond; and
the dashed line marked with the asterisk indicates attachment to a moiety 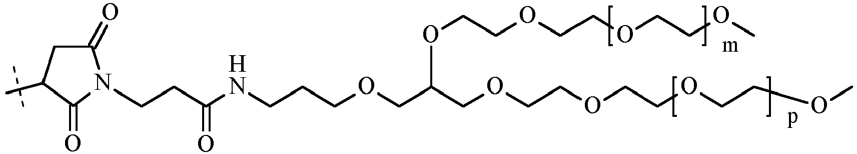 wherein
m and p are independently an integer ranging from and including 400 to 500.

2. The PTH conjugate for use of claim 1, wherein the starting dose ranges from 2.9 to 4.5 nmol/day.

3. The PTH conjugate for use of claim 1, wherein the starting dose ranges from 3.6 to 4.4 nmol/day.

4. The PTH conjugate for use of claim 1, wherein the starting dose ranges from 0.8 to 3.9 nmol/day.

5. The PTH conjugate for use of claim 1 or 4, wherein the starting dose ranges from 1.5 to 3.4 nmol/day.

6. The PTH conjugate for use of any one of claims 1, 4 or 5, wherein the starting dose is 2.9 ± 0.3 nmol/day.

7. The PTH conjugate for use of claim 1, wherein the starting dose is 4.4 ± 0.3 nmol/day.

8. The PTH conjugate for use of claim 1 or 7, wherein the starting dose is 4.4 ± 0.2 nmol/day.

9. The PTH conjugate for use of any one of claims 1, 7 or 8, wherein the starting dose is 4.4 ± 0.1 nmol/day.

10. The PTH conjugate for use of any one of claims 1 to 9, wherein the starting dose is administered to a patient as one subcutaneous injection.

11. The PTH conjugate for use of any one of claims 1 to 10, wherein the starting dose is administered with a pen injector.

## Patentansprüche

1. Ein PTH-Konjugat, in dem ein PTH-Rest reversibel konjugiert ist an einen polymeren Rest, oder ein pharmazeutisch unbedenkliches Salz davon oder eine pharmazeutische Zusammensetzung enthaltend besagtes PTH-Konjugat oder pharmazeutisch unbedenkliche Salz davon zur Verwendung in der Behandlung von Hypoparathyreoidismus bei einem menschlichen Patienten, wobei die Anfangsdosis im Bereich von 0.8 bis 4.9 nmol/Tag liegt und wobei das PTH-Konjugat gemäß der Formel (IIf-i) ist: 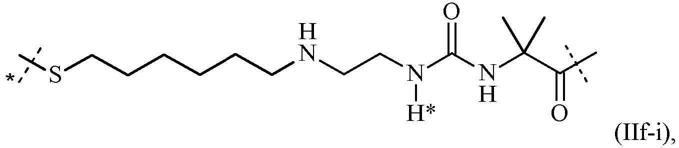 wobei
die unmarkierte gestrichelte Linie die Anknüpfung an die N-terminale funktionelle Aminogruppe eines PTH-Rests mit der Sequenz der SEQ ID NO:51 durch Bildung einer Amidbindung anzeigt; und
die mit dem Asterisk markierte gestrichelte Linie Anknüpfung an einen Rest 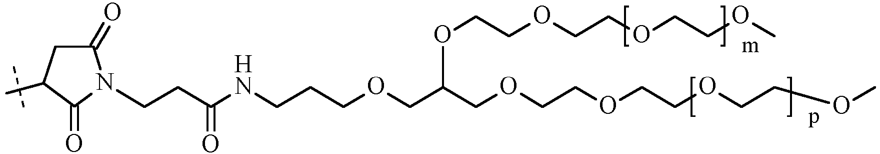
anzeigt,
wobei
m und p unabhängig eine ganze Zahl im Bereich von und einschließlich 400 bis 500 sind.

2. Das PTH-Konjugat zur Verwendung nach Anspruch 1, wobei die Anfangsdosis im Bereich von 2.9 bis 4.5 nmol/Tag liegt.

3. Das PTH-Konjugat zur Verwendung nach Anspruch 1, wobei die Anfangsdosis im Bereich von 3.6 bis 4.4 nmol/Tag liegt.

4. Das PTH-Konjugat zur Verwendung nach Anspruch 1, wobei die Anfangsdosis im Bereich von 0.8 bis 3.9 nmol/Tag liegt.

5. Das PTH-Konjugat zur Verwendung nach Anspruch 1 oder 4, wobei die Anfangsdosis im Bereich von 1.5 bis 3.4 liegt.

6. Das PTH-Konjugat zur Verwendung nach einem der Ansprüche 1, 4 oder 5, wobei die Anfangsdosis 2.9 ± 0.3 nmol/Tag ist.

7. Das PTH-Konjugat zur Verwendung nach Anspruch 1, wobei die Anfangsdosis 4.4 ± 0.3 nmol/Tag ist.

8. Das PTH-Konjugat zur Verwendung nach Anspruch 1 oder 7, wobei die Anfangsdosis 4.4 ± 0.2 nmol/Tag ist.

9. Das PTH-Konjugat zur Verwendung nach einem der Ansprüche 1, 7 oder 8, wobei die Anfangsdosis 4.4 ± 0.1 nmol/Tag ist.

10. Das PTH-Konjugat zur Verwendung nach einem der Ansprüche 1 bis 9, wobei die Anfangsdosis dem Patienten als eine subkutane Injektion gegeben wird.

11. Das PTH-Konjugat zur Verwendung nach einem der Ansprüche 1 bis 10, wobei die Anfangsdosis mit einem Pen-Injektor gegeben wird.

## Revendications

1. Conjugué PTH, dans lequel un groupement PTH est conjugué de manière réversible à un groupement polymérique ou un sel pharmaceutiquement acceptable de celui-ci ou une composition pharmaceutique comprenant ledit conjugué PTH ou un sel pharmaceutiquement acceptable de celui-ci pour une utilisation dans le traitement de l'hypoparathyroïdie chez un patient humain, dans lequel la dose de départ est dans la plage de 0,8 à 4,9 nmole/jour et dans lequel le conjugué PTH est de formule (IIf-i) : 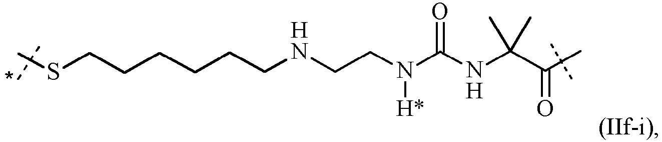 dans laquelle
la ligne pointillée non marquée indique la fixation au groupe fonctionnel amine N-terminal d'un groupement PTH ayant la séquence SEQ ID NO:51 par formation d'une liaison amide ; et
la ligne pointillée marquée d'un astérisque indique la fixation à un groupement 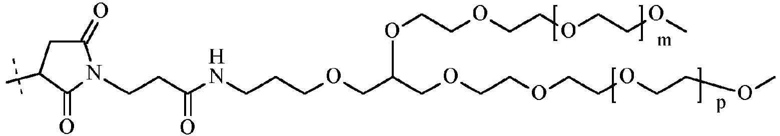
dans lequel
m et p sont indépendamment un entier dans la plage de 400 à 500 inclus.

2. Conjugué PTH pour une utilisation selon la revendication 1, dans lequel la dose de départ est dans la plage de 2,9 à 4,5 nmole/jour.

3. Conjugué PTH pour une utilisation selon la revendication 1, dans lequel la dose de départ est dans la plage de 3,6 à 4,4 nmole/jour.

4. Conjugué PTH pour une utilisation selon la revendication 1, dans lequel la dose de départ est dans la plage de 0,8 à 3,9 nmole/jour.

5. Conjugué PTH pour une utilisation selon la revendication 1 ou 4, dans lequel la dose de départ est dans la plage de 1,5 à 3,4 nmole/jour.

6. Conjugué PTH pour une utilisation selon l'une quelconque des revendications 1, 4 ou 5, dans lequel la dose de départ est de 2,9 ± 0,3 nmole/jour.

7. Conjugué PTH pour une utilisation selon la revendication 1, dans lequel la dose de départ est de 4,4 ± 0,3 nmole/jour.

8. Conjugué PTH pour une utilisation selon la revendication 1 ou 7, dans lequel la dose de départ est de 4,4 ± 0,2 nmole/jour.

9. Conjugué PTH pour une utilisation selon l'une quelconque des revendications 1, 7 ou 8, dans lequel la dose de départ est de 4,4 ± 0,1 nmole/jour.

10. Conjugué de PTH pour une utilisation selon l'une quelconque des revendications 1 à 9, dans lequel la dose de départ est administrée à un patient sous la forme d'une injection sous-cutanée.

11. Conjugué de PTH pour une utilisation selon l'une quelconque des revendications 1 à 10, dans lequel la dose de départ est administrée avec un stylo injecteur.
